(19)

Europäisches
Patentamt
European
Patent Office
Office européen
des brevets

(11) **EP 2 327 990 A1**

(12) **EUROPEAN PATENT APPLICATION**

(43) Date of publication:
**01.06.2011 Bulletin 2011/22**

(51) Int Cl.:
***G01N 37/00*** *(2006.01)*

(21) Application number: **11156208.8**

(22) Date of filing: **20.11.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **20.11.2006 US 860453 P**

(62) Document number(s) of the earlier application(s) in accordance with Art. 76 EPC:
**07862180.2 / 2 109 773**

(71) Applicant: **Nativis, Inc**
**San Diego, CA 92121 (US)**

(72) Inventor: **The designation of the inventor has not yet been filed**

(74) Representative: **Vossius & Partner**
**Siebertstrasse 4**
**81675 München (DE)**

Remarks:
This application was filed on 28-02-2011 as a divisional application to the application mentioned under INID code 62.

(54) **Apparatus and method for transducing an in vitro or mammalian system with a low-frequency signal**

(57)     Method and apparatus for generating and selecting low-frequency time-domain signals capable of transducing a mammalian system, to produce an agent-specific effect on the system. Low-frequency time-domain signals are generated in the presence of an injected magnetic stimulus, and the resulting signals are selected by a scoring algorithm, and optionally, by testing each signal identified by the scoring algorithm for its ability to produce an agent-specific response in an in vitro system containing components that are responsive to the agent. The selected signals are used to transduce the mammalian system by applying the signals to an electromagnetic transduction coil that holds the sample.

*FIG. 7*

**EP 2 327 990 A1**

**Description**

Field of the Invention

The present invention relate to signals readable by a system for converting or transducing the signal into electromagnetic waves, and to methods of producing and applying such signals.

Background of the Invention

One of the accepted paradigms in the fields of chemistry and biochemistry is that chemical or biochemical effector agents, e.g., molecules, interact with target systems through various physicochemical forces, such as ionic, charge, or dispersion forces or through the cleavage or formation of covalent or charge-induced bonds. These forces may involve energy modes in either the effector agent or target system.

A corollary of this paradigm is the requirement, in effector-target systems, of the effector agent in the target environment. However, what is not known or understood is whether this requirement is related to the actual presence of the effector, or whether it may be due, at least as to certain effector functions, to the presence of energetic modes that are characteristic of the effector. If effector function can be simulated, at least in part, by certain characteristic energetic modes, it may be possible to "simulate" the effect of the effector agent in a target system by exposing the system to certain energetic modes that are characteristic of the effector. If so, the questions that naturally arise are: what effector-molecule energy modes are effective, how can they be converted or transduced into the form of measurable signals, and how can these signals be used to effect a target system, that is, mimic at least some of the effector functions of the molecule in a target system?

These questions were addressed in recently filed co-owned patent applications 60/593,006 and 60/591,549 (attorney docket numbers 38547-8010 and -8011). Experiments conducted in support of the invention described in the application demonstrate that certain effector functions on a target system (in this case, one of a number of biological systems), can be duplicated by exposing the target system to electromagnetic waves produced by "transducing" a time-domain signal of the effector compound. According to the earlier-described invention, the time-domain signal is produced by recording a signal produced by the compound in a shielded environment, while injecting a Gaussian white noise stimulus into the recording apparatus at a level that enhances the ability to observe low-frequency stochastic events produced by the compound. In the earlier-described application, the transducing signal was the actual compound time-domain signal of the effector compound.

The possibility of achieving effector-molecule functions by exposing a target system to characteristic effector-molecule signals, without the need for the actual presence of the effector agent, has a number of important and intriguing applications. Instead of treating an organism by the application of a drug, the same effect may be achieved by exposing the organism to drug-specific signals. In the field of nanofabrication, it might now be possible to catalyze or encourage self-assembly patterns by introducing in the assembly system, signals characteristic of multivalent effector molecules capable of promoting the desired pattern of self-assembly.

It would be desirable, therefore, to employ systematic methods for producing and selecting low-frequency time domain signals that are effective, in a magnetic transduction environment, of producing agent-specific effects on a mammalian or *in vitro* system.

Summary of the invention

The invention includes, in one aspect, a method for generating a signal capable of producing an agent-specific effect on a mammalian system, when the system is transduced by the signal within the environment of an electromagnetic tranducer. The method includes the steps of:

(a) placing a sample containing the agent in a sample container having both magnetic and electromagnetic shielding, where the sample acts as a signal source for low-frequency molecular signals, and where the magnetic shielding is external to a cryogenic container;
(b) injecting a stimulus magnetic field into the sample, under a selected stimulus magnetic-field condition,
(c) recording a low-frequency, time-domain signal composed of sample source radiation superimposed on the injected stimulus magnetic field in the cryogenic container,
(d) repeating steps (b) and (c) at each of a plurality of different stimulus magnetic field conditions,
(e) identifying from among the signals recorded in step (c), one or more signals having the highest signal scores when analyzed by a scoring algorithm that measures the number of low-frequency components above a given threshold in a recorded signal,
(f) testing each signal identified in step (e) for its ability to produce an agent-specific response in a *in vitro* system containing components that are responsive to the agent, when the *in vitro* system is transduced with the signal within the environment of an electromagnetic tranducer, and

(g) selecting one or more signals that produce the greatest agent-specific transduction effect in the *in vitro* system.

The different conditions of stimulus magnetic field may include (i) white noise, injected at a voltage level calculated to produce a selected magnetic field at the sample of between 0 and 1 G (Gauss), (ii) a DC offset, injected at a voltage level calculated to produce a selected magnetic field at the sample of between 0 and 1 G, and (iii) sweeps over a low-frequency range, injected successively over a sweep range between at least about 0-1kHz, and at an injected voltage calculated to produce a selected magnetic field at the sample of between 0 and 1 G.

Step (f) in the method may further include, after testing a time-domain signal for its ability to produce an agent-specific response in a *in vitro* system containing components that are responsive to the agent, testing the ability of signal to produce an agent-specific response under varying transduction conditions, including variations in transduction voltage applied within the environment of an electromagnetic tranducer, thus to optimize transduction conditions for transduction in the mammalian system.

The step of identifying from among the recorded signals, one or more signals having the highest signal scores, may be carried out by one of the following algorithmic scoring methods:

(i) autocorrelating the time domain signal, generating an FFT (Fast Fourier Transform) of the autocorrelated signal over a selected frequency range within the range DC to 8kHz, assigning to the FFT signal a score related to a number of peaks above a mean average noise value, and selecting a time-domain signal based on the score;

(ii) calculating a pair of phase spaces for two time domain signals, and performing a mathematical comparison to provide a measure of difference between the two;

(iii) generating a histogram that shows, for each event bin f over a selected frequency range within a range DC to 8kHz, a number of event counts in each bin, where f is a sampling rate for sampling the time domain signal, assigning to the histogram, a score related to number of bins that are above a given threshold; and selecting a time-domain signal based on the score;

(iv) cross-correlating a small block of data near the beginning of the time domain signal with the remainder of the time series, and counting the occurrences that the resulting cross-correlation surpasses a given threshold; and

(v) calculating a series of Fourier spectra of the time-domain signal over each of multiple defined time periods, in a selected frequency range between DC and 8 kHz, averaging the Fourier spectra; assigning to the averaged FFT signal a score related to the number of peaks above a mean average noise value, and selecting a time-domain signal based on the score.

The electromagnetic transducer employed in the method may include a Helmholtz coil having a pair of aligned electromagnetic coils defining a sample magnetic environment therebetween, and the step of testing each signal identified for its ability to produce an agent-specific response in an *in vitro* system may include placing the *in vitro* system within the aligned coils, and transducing the system with an agent-specific time-domain signal identified in step (e).

Where the agent is an anti-neoplastic drug effective to promote tubulin aggregation *in vitro*, step (f) of the method may include placing a tubulin-containing composition within the environment of the electromagnetic transducer, and transducing the composition with an agent-specific time-domain signal identified in step (e).

In another aspect, the invention includes a method for generating signals capable of producing an agent-specific effect on a *in vitro* or mammalian system when the system is transduced by the signal within the environment of an electromagnetic transducer. The method includes the steps of:

(a) placing a sample containing the agent in a sample container having both magnetic and electromagnetic shielding, wherein the sample acts as a signal source for molecular signals, and wherein the magnetic shielding is external to a cryogenic container;

(b) injecting a stimulus magnetic field into the sample, a under selected stimulus magnetic field condition selected from the group consisting of (i) white noise, injected at a voltage level calculated to produce a selected magnetic field at the sample of between 0 and 1 G (Gauss), (ii) a DC offset, injected at a voltage level calculated to produce a selected magnetic field at the sample of between 0 and 1 G, and (iii) sweeps over a low-frequency range, injected successively over a sweep range between at least about 0-1 kHz, and at an injected voltage calculated to produce a selected magnetic field at the sample of between 0 and 1 G.

(c) recording a low-frequency, time-domain signal composed of sample source radiation superimposed on the injected stimulus magnetic field in the cryogenic container,

(d) repeating steps (b) and (c) at each of a plurality of different stimulus magnetic field conditions,

(e) identifying from among the signals recorded in step (c), one or more signals having the highest signal scores when analyzed by a scoring algorithm that measures the number of low-frequency components above a given threshold in a recorded signal, and

(f) transducing the *in vitro* or mammalian system by placing the system within the environment of an electromagnetic transducer, and transducing the sample with a signal identified in step (e).

Step (e) of the method may be carried out, for example, by autocorrelating the time domain signal, generating an FFT (Fast Fourier Transform) of the autocorrelated signal over a selected frequency range within the range DC to 8kHz, assigning to the FFT signal a score related to a number of peaks above a mean average noise value, and selecting a time-domain signal based on the score;

The electromagnetic transducer employed in the method may include a Helmholtz coil having a pair of aligned electromagnetic coils defining an exposure station therebetween, constituting the environment of the electromagnetic environment, and step (f) of the method may include placing the chemical or *in vitro* system within the aligned coils, and transducing the system with an agent-specific time-domain signal identified in step (e).

Where the agent is an anti-neoplastic drug effective to promote tubulin aggregation in the *in vitro* system, step (f) may include includes placing a tubulin-containing composition within the environment of the electromagnetic transducer, and transducing the composition with an agent-specific, time-domain signal identified in step (e) under conditions effective to produce signal-dependent aggregation of the tubulin in the composition.

In still another embodiment, the invention includes apparatus for producing low-frequency, time-domain signals that are candidates for transducing a *in vitro* or mammalian system that is responsive to the presence of a selected agent. The apparatus includes:

(a) a sample container adapted for receiving a sample of an agent, the container having both magnetic and electromagnetic shielding, where the sample acts as a signal source for molecular signals, and where the magnetic shielding is external to a cryogenic container;

(b) an adjustable-power source operable to inject a stimulus magnetic field into the container, with a sample in the container, at each of a plurality of selected stimulus magnetic field conditions selected from the group consisting of (i) white noise, injected at a voltage level calculated to produce a selected magnetic field at the sample of between 0 and 1 G (Gauss), (ii) a DC offset, injected at a voltage level calculated to produce a selected magnetic field at the sample of between 0 and 1 G, and (iii) sweeps over a low-frequency range, injected successively over a sweep range between at least about 0-1kHz, and at an injected voltage calculated to produce a selected magnetic field at the sample of between 0 and 1 G.

(c) a detector for recording, at each of the different stimulus magnetic field conditions injected by said power source, (b) the electromagnetic time-domain signals composed of sample source radiation superimposed on the injected stimulus magnetic fields,

(d) a memory device for storing the signals recorded by the detector, and

(e) a computer operable to:

(i) retrieve time-domain signals stored in the memory device;

(ii) analyzing the retrieved time-domain signals by a scoring algorithm that measures the number of low-frequency components above a given threshold in a recorded signal, and

(iii) identifying those time-domain signals having the greatest number of low-frequency components above the threshold.

The sample container may be an attenuation tube having a sample-holding region, a magnetic shielding cage surrounding the region, and a Faraday cage contained within the magnetic shielding cage and also surrounding the region, the source of a Gaussian noise includes a Gaussian noise generator and a Helmholtz coil which is contained within the magnetic cage and the Faraday cage, and which receives a noise output signal from the noise generator, and which further includes, for use in removing stationary noise components in the time-dependent signal, a signal inverter operatively connected to the noise source and to the SQUID (Superconducting QUantum interference Device), for receiving Gaussian noise from the noise source and outputting into the SQUID, Gaussian noise in inverted form with respect to the Gaussian noise injected into the sample.

The power source may be operable to inject an offset voltage into the container, with a sample in the container, at each of a plurality of selected offset voltages calculated to produce a selected magnetic field at the sample of between 0 and 1 G (Gauss). Alternatively, the power source may be operable to inject generate successive sweeps over a sweep-frequency range between at least about 0 and 1 kHz, at each of a plurality of different sweep voltages calculated to produce a selected magnetic field at the sample of between 0 and 1 G (Gauss).

The computer in the apparatus may be operable, in analyzing the retrieved time-domain signals is operable to apply an analysis algorithm selected from one of:

(i) autocorrelating the time domain signal, generating an FFT (Fast Fourier Transform) of the autocorrelated signal over a selected frequency range within the range DC to 8kHz, assigning to the FFT signal a score related to a number of peaks above a mean average noise value, and selecting a time-domain signal based on the score;

(ii) calculating a pair of phase spaces for two time domain signals, and performing a mathematical comparison to provide a measure of difference between the two;

(iii) generating a histogram that shows, for each event bin f over a selected frequency range within a range DC to 8kHz, a number of event counts in each bin, where f is a sampling rate for sampling the time domain signal, assigning to the histogram, a score related to number of bins that are above a given threshold; and selecting a time-domain signal based on the score;

(iv) cross-correlating a small block of data near the beginning of the time domain signal with the remainder of the time series, and counting the occurrences that the resulting cross-correlation surpasses a given threshold; and

(v) calculating a series of Fourier spectra of the time-domain signal over each of multiple defined time periods, in a selected frequency range between DC and 8 kHz, averaging the Fourier spectra; assigning to the averaged FFT signal a score related to the number of peaks above a mean average noise value, and selecting a time-domain signal based on the score.

Also disclosed is a system for producing an agent-specific effect on a mammalian system. The system includes:

(1) a storage medium having stored thereon, an agent-specific low-frequency time-domain signal produced by the steps of:

(a) placing a sample to which the mammalian system is responsive in a sample container having both magnetic and electromagnetic shielding, wherein the sample acts as a signal source for low-frequency molecular signals, and wherein the magnetic shielding is external to a cryogenic container;

(b) injecting a stimulus magnetic field into the sample, under a selected stimulus magnetic field condition,

(c) recording a low-frequency, time-domain signal composed of sample source radiation superimposed on the injected stimulus magnetic field in the cryogenic container,

(d) repeating steps (b) and (c) at each of a plurality of different stimulus magnetic field conditions,

(e) identifying from among the signals recorded in step (c), one or more signals having the highest signal scores when analyzed by a scoring algorithm that measures the number of low-frequency components above a given threshold in a recorded signal,

(f) testing each signal identified in step (e) for its ability to produce an agent-specific response in an *in vitro in vitro* system containing components that are responsive to the agent, when the *in vitro* system is transduced by the signal within the environment of an electromagnetic transducer,

(2) an electromagnetic transducer composed of one or more electromagnetic coils, said coils having an interior region defining a magnetic environment in which the sample is received, and

(3) an amplifier for amplifying a signal from the storage medium and supplying the amplified signal to the transduction coil(s).

The electromagnetic transducer may include a Helmholtz coil having a pair of aligned electromagnetic coils defining an interior region therebetween.

In yet another embodiment, the invention includes a storage medium having stored thereon, an agent-specific low-frequency time-domain signal produced by the steps of:

(a) placing a sample to which the mammalian system is responsive in a sample container having both magnetic and electromagnetic shielding, wherein the sample acts as a signal source for low-frequency molecular signals, and wherein the magnetic shielding is external to a cryogenic container;

(b) injecting a stimulus magnetic field into the sample, under a selected stimulus magnetic field condition,

(c) recording a low-frequency, time-domain signal composed of sample source radiation superimposed on the injected stimulus magnetic field in the cryogenic container,

(d) repeating steps (b) and (c) at each of a plurality of different stimulus magnetic field conditions,

(e) identifying from among the signals recorded in step (c), one or more signals having the highest signal scores when analyzed by a scoring algorithm that measures the number of low-frequency components above a given threshold in a recorded signal,

(f) testing each signal identified in step (e) for its ability to produce an agent-specific response in an *in vitro in vitro* system containing components that are responsive to the agent, when the *in vitro* system is transduced by the signal within the environment of an electromagnetic transducer.

The signal carried on the storage medium may be produced, for example, by an anti-neoplastic agent effective to promote tubulin aggregation *in vitro.*

These and other objects and features of the invention will be more fully understood when the following detailed description of the invention is read in conjunction with the accompanying drawings.

Brief Description of the Drawings

Figure 1 is an isometric view of one embodiment of a molecular electromagnetic signaling detection apparatus formed in accordance with one embodiment of the present invention;

Figure 2 is an enlarged, detail view of the faraday cage and its contents shown in Figure 1;

Figure 3 is an enlarged, cross sectional view of one of the attenuation tubes shown in Figures 1 and 2.

Figure 4 is a cross-section view of the faraday cage and its contents shown in Figure 2.

Figure 5 is a diagram of an alternative electromagnetic emission detection system.

Figure 6 diagram of the processing unit included in the detection system of the above figures.

Figure 7 is a diagram of an alternative processing unit to that of Figure 6.

Figure 8 is a flow diagram of the signal detection and processing performed by the present system.

Figure 9 shows a high-level flow diagram of data flow for the histogram spectral plot method of the invention;

Figure 10 is a flow diagram of the algorithm for generating a spectral plot histogram, in accordance with the invention.

Figure 11 is a flow diagram of steps in identify optimal time-domain signals in accordance with a second embodiment of the method of the invention;

Figure 12 is a flow diagram of steps to identify optimal time-domain signals in accordance with a third embodiment of the method of the invention;

Figure 13 shows an example signal *Score* result, where the upper graph shows *File #* on the X -axis, *Tau* on the Y-axis, and *Score* on the Z-axis.

Figure 14 shows the transduction equipment layout in a typical transduction experiment.

Figure 15 shows a transduction coil and container used in a typical transduction experiment.

Figures 16A-16F are bar-graphs of the rate of tubulin polymerization measured at OD, calculated at 1, 2, 3, 4, and 5, minutes, respectively, after addition of taxol or initiation of a transduction signal;

Fig. 17 is a bar graph showing Vmax values for the tubulin assay of Figure 16, calculated at the end of a 20-minute assay reaction; and

Fig. 18 shows survival time in days, for mice injected intracranially with glioblastoma cells, and after transduction with a taxol time-domain signal.

Detailed Description of the Intention

I. Definitions

The terms below have the following definitions unless indicated otherwise.

"Magnetic shielding" refers to shielding that decreases, inhibits or prevents passage of magnetic flux as a result of the magnetic permeability of the shielding material.

"Electromagnetic shielding" refers to, e.g., standard Faraday electromagnetic shielding, or other methods to reduce passage of electromagnetic radiation.

"Time-domain signal" or 'time-series signal" refers to a signal with transient signal properties that change over time.

"Sample-source radiation" or refers to magnetic flux or electromagnetic flux emissions resulting from molecular motion of a sample, such as the rotation of a molecular dipole in a magnetic field. Because sample source radiation is produced in the presence of an injected magnetic-field stimulus," it is also referred to as "sample source radition superimposed on injected magnetic field stimulus."

"Stimulus magnetic field" or "Magnetic-field stimulus" refers to a magnetic field produced by injecting (applying) to magnetic coils surrounding a sample, one of a number of electromagnetic signals that may include (i) white noise, injected at voltage level calculated to produce a selected magnetic field at the sample of between 0 and 1 G (Gauss), (ii) a DC offset, injected at voltage level calculated to produce a selected magnetic field at the sample of between 0 and 1 G, and (iii) sweeps over a low-frequency range, injected successively over a sweep range between at least about 0-1 kHz, and at an injected voltage calculated to produce a selected magnetic field at the sample of between 0 and 1 G. The magnetic field produced at the sample may be readily calculated using known electromagnetic relationships, knowing the shape and number of windings in the injection coil, the voltage applied to coils, and the distance between the injection coils and the sample.

A "selected stimulus magnetic-field condition" refers to a selected voltage applied to a white noise or DC offset signal, or a selected sweep range, sweep frequency and voltage of an applied sweep stimulus magnetic field.

"White noise" means random noise or a signal having simultaneous multiple frequencies, e.g. white random noise or deterministic noise. "Gaussian white noise" means white noise having a Gaussian power distribution. "Stationary Gaussian white noise" means random Gaussian white noise that has no predictable future components. "Structured noise" is white noise that may contain a logarithmic characteristic which shifts energy from one region of the spectrum to another, or it may be designed to provide a random time element while the amplitude remains constant. These two represent pink and uniform noise, as compared to truly random noise which has no predictable future component. "Uniform noise" means white noise having a rectangular distribution rather than a Gaussian distribution.

"Frequency-domain spectrum" refers to a Fourier frequency plot of a time-domain signal.

"Spectral components" refer to singular or repeating qualities within a time-domain signal that can be measured in the frequency, amplitude, and/or phase domains. Spectral components will typically refer to signals present in the frequency domain.

"Faraday cage" refers to an electromagnetic shielding configuration that provides an electrical path to ground for unwanted electromagnetic radiation, thereby quieting an electromagnetic environment.

A "signal-analysis score" refers to a score based on the number and/or amplitude of agent-specific spectral peaks observed over a selected low-frequency range, e.g., DC to 1 kHz or DC to 8 kHz, in a time-domain signal recorded for an agent or sample that has been processed by a suitable method, such as one of the five methods described herein, to reveal identifiable spectral features that are specific to the agent or sample.

An "optimized agent-specific time-domain signal" refers to a time-domain signal having a maximum or near-maximum signal-analysis score.

*"In vitro* system" refers to a biochemical system having of one or more biochemical components, such as nucleic acid or protein components, including receptors and structural proteins isolated or derived from a virus, bacteria, or multicellular plant or animal. An *in vitro* system typically is a solution or suspension of one or more isolated or partially isolated *in vitro* components in an aqueous medium, such as a physiological buffer. The term also refers to a cell culture system containing bacterial or eukaryotic cells in a culture medium.

"Mammalian system" refers to a mammal, include a laboratory animal such as mouse, rat, or primate that may serve as a model for a human disease, or a human patient.

"Agent-specific effect" refers to an effect observed when an *in vitro* or mammalian system is exposed to an agent (effector). Examples of agent-specific *in vitro* effects include, for example, a change in the state of aggregation of components of the system, the binding the an agent to a target, such as a receptor, and the change in growth or division of cells in culture.

II. Recording apparatus and method

The following description of a signal recording devices in accordance with the invention provides specific details for a thorough understanding of, and enabling description for, embodiments of the invention. However, one skilled in the art will understand that the invention may be practiced without these details. In other instances, well-known structures and functions have not been shown or described in detail to avoid unnecessarily obscuring the description of embodiments of the invention.

As explained in detail below, embodiments of the present invention are directed to providing an apparatus and method for the repeatable detection and recording of low-threshold molecular electromagnetic signals for later, remote use. A magnetically shielded faraday cage shields the sample material and detection apparatus from extraneous electromagnetic signals. Within the magnetically shielded faraday cage, a coil injects a stimulus signal such as Gaussian white noise, a non-ferrous tray holds the sample, and a gradiometer detects low-threshold molecular electromagnetic response signals. The apparatus further includes a superconducting quantum interference device ("SQUID") and a preamplifier.

The apparatus is used by placing a sample within the magnetically shielded faraday cage in close proximity to the coil that generates the stimulus signal and the gradiometer that measures the response. A stimulus signal is injected through the stimulus coil and modulated until the molecular electromagnetic signal is optimized. The molecular electromagnetic response signal, shielded from external interference by the faraday cage and the field generated by the stimulus coil, is then detected and measured by the gradiometer and SQUID. The signal is then amplified and transmitted to any appropriate recording or measuring equipment.

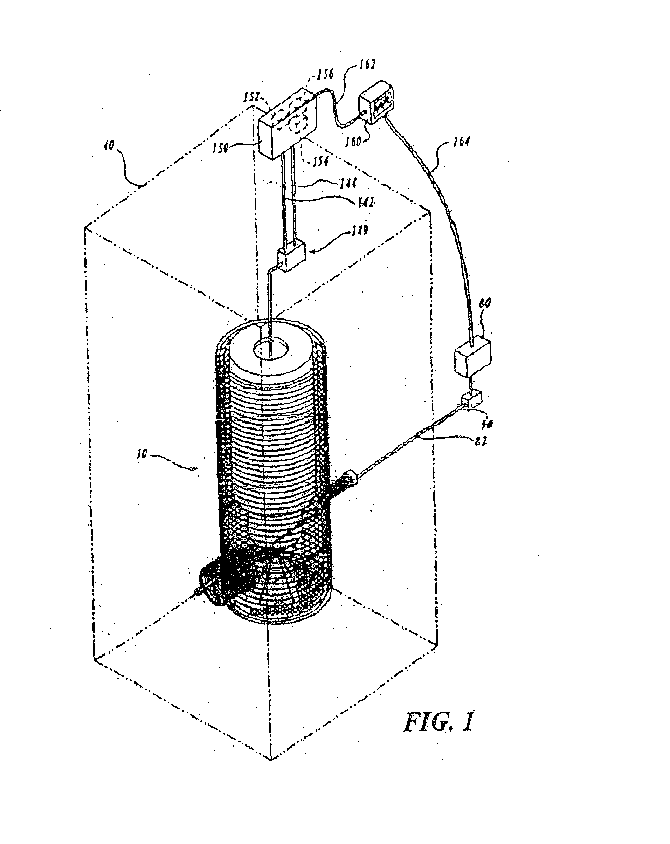

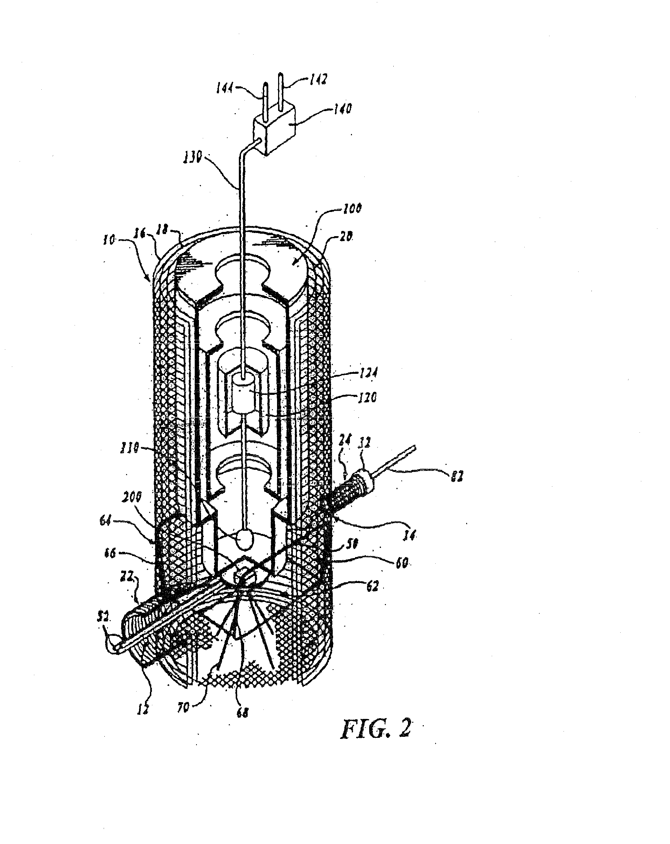

Referring to Figures 1 and 2, there is shown a shielding structure 10, in the form of a Faraday cage, which includes, in an outer to inner direction, a conductive wire cage 16 which is a magnetic shield and inner conductive wire cages 18 and 20 which provide electromagnetic shielding. In another embodiment, the outer magnetic shield is formed of a solid aluminum plate material having an aluminum-nickel alloy coating, and the electromagnetic shielding is provided by two inner wall structures, each formed of solid aluminum.

The faraday cage 10 is open at the top, and includes side openings 12 and 14. The faraday cage 10 is further comprised of three copper mesh cages 16, 18 and 20, nestled in one another. Each of the copper mesh cages 16, 18 and 20 is electrically isolated from the other cages by dielectric barriers (not shown) between each cage.

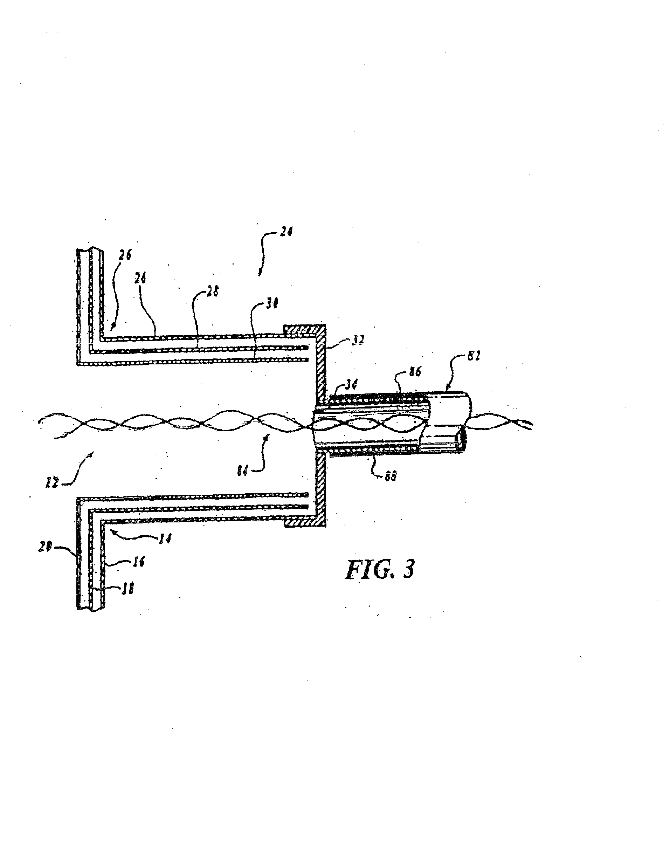

Side openings 12 and 14 further comprise attenuation tubes 22 and 24 to provide access to the interior of the faraday cage 10 while isolating the interior of the cage from external sources of interference. Referring to Figure 3, attenuation tube 24 is comprised of three copper mesh tubes 26, 28 and 30 (Fig. 3), nestled in one another. The exterior copper mesh cages 16,18 and 20 are each electrically connected to one of the copper mesh tubes 26, 28 and 30, respectively. Attenuation tube 24 is further capped with cap 32, with the cap having hole 34. Attenuation tube 22 is similarly comprised of copper mesh tubes 26, 28 and 30, but does not include cap 32.

Referring again to Figure 2, a low-density nonferrous sample tray 50 is mounted in the interior of the faraday cage 10. The sample tray 50 is mounted so that it may be removed from the faraday cage 10 through the attenuation tube 22 and side opening 12. Three rods 52, each of which is greater in length than the distance from the center vertical axis of the faraday cage 10 to the outermost edge of the attenuation tube 22, are attached to the sample tray 50. The three rods 52 are adapted to conform to the interior curve of the attenuation tube 22, so that the sample tray 50 may be positioned in the center of the faraday cage 10 by resting the rods in the attenuation tube. In the illustrated embodiment, the sample tray 50 and rods 52 are made of glass fiber epoxy. It will be readily apparent to those skilled in the art that the sample tray 50 and rods 52 may be made of other nonferrous materials, and the tray may be mounted in the faraday cage 10 by other means, such as by a single rod.

Referring again to Figure 2, mounted within the Faraday cage 10 and above the sample tray 50 is a cryogenic dewar 100. In the disclosed embodiment, the dewar 100 is adapted to fit within the opening at the top of faraday cage 10 and is a Model BMD-6 Liquid Helium Dewar manufactured by Tristan Technologies, Inc. The dewar 100 is constructed of a glass-fiber epoxy composite. A gradiometer 110 with a very narrow field of view is mounted within the dewar 100 in position so that its field of view encompasses the sample tray 50. In the illustrated embodiment, the gradiometer 110 is a first order axial detection coil, nominally 1 centimeter in diameter, with a 2 % balance, and is formed from a superconductor. The gradiometer can be any form of gradiometer excluding a planar gradiometer. The gradiometer 110 is connected to the input coil of one low temperature direct current superconducting quantum interference device ("SQUID") 120. In the disclosed embodiment, the SQUID is a Model LSQ/20 LTS dc SQUID manufactured by Quantum Design, Inc. It will be recognized by those skilled in the art that high temperature or alternating current SQUIDs can be used without departing from the spirit and scope of the invention. In an alternative embodiment, the SQUID 120 includes a noise suppression coil 124.

The disclosed combination of gradiometer 110 and SQUID 120 have a sensitivity of 5 microTesla/$\sqrt{\text{Hz}}$ when measuring magnetic fields.

The output of SQUID 120 is connected to a Model SP Cryogenic Cable 130 manufactured by Tristan Technologies, Inc. The Cryogenic Cable 134 is capable of withstanding the temperatures within and without the dewar 100 and transfers the signal from the SQUID 120 to Flux-Locked Loop 140, which is mounted externally to the faraday cage 10 and dewar 100. The Flux-Locked Loop 140 in the disclosed embodiment is an iFL-301-L Flux Locked Loop manufactured by Tristan Technologies, Inc.

Referring to Figure 1, the Flux Locked Loop 140 further amplifies and outputs the signal received from the SQUID 120 via high-level output circuit 142 to an iMC-303 iMAG® SQUID controller 150. The Flux-Locked Loop 140 is also connected via a model CC-60 six-meter fiber-optic composite connecting cable 144 to the SQUID controller 150. The fiber-optic connecting cable 144 and SQUID controller 150 are manufactured by Tristan Technologies, Inc. The controller 150 is mounted externally to the magnetic shielding cage 40. The fiber-optic connecting cable 144 carriers control signals from the SQUID controller 150 to the Flux Locked Loop 140, further reducing the possibility of electromagnetic interference with the signal to be measured. It will be apparent to those skilled in the art that other Flux-Locked Loops, connecting cables, and SQUID controllers can be used without departing from the spirit and scope of the invention.

The SQUID controller 150 further comprises high resolution analog to digital converters 152, a standard GP-IB bus 154 to output digitalized signals, and BNC connectors 156 to output analog signals. In the illustrated embodiment, the BNC connectors are connected to a dual trace oscilloscope 160 through patch cord 162.

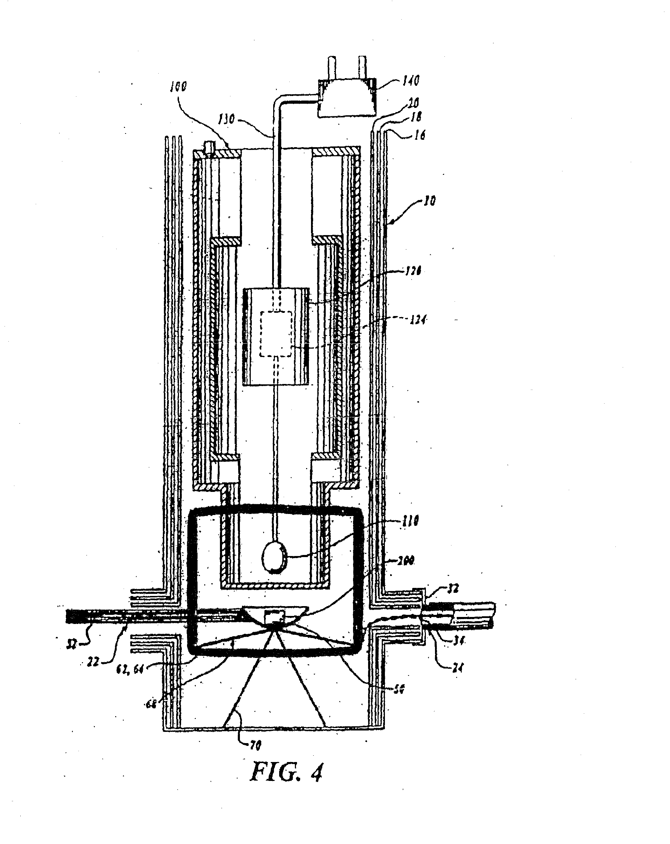

Referring to Figures 2 and 4 a two-element Helmholtz transformer 60 is installed to either side of the sample tray 50 when the sample tray is fully inserted within the faraday cage 10. In the illustrated embodiment, the coil windings 62 and 64 of the Helmholtz transformer 60 are designed to operate in the direct current to 50 kilohertz range, with a center frequency of 25 kilohertz and self-resonant frequency of 8.8 megahertz. In the illustrated embodiment, the coil windings 62 and 64 are generally rectangular in shape and are approximately 8 inches tall by 4 inches wide. Other Helmholtz coil shapes may be used but should be shaped and sized so that the gradiometer 110 and sample tray 50 are positioned within the field produced by the Helmholtz coil. Each of coil windings 62 and 64 is mounted on one of two low-density nonferrous frames 66 and 68. The frames 66 and 68 are hingedly connected to one another and are supported by legs 70. Frames 66 and 68 are slidably attached to legs 70 to permit vertical movement of the frames in relation to the lower portion of dewar 100. Movement of the frames permits adjustment of the coil windings 62 and 64 of the Helmholtz transformer 60 to vary the amplitude of the magnetic-field stimulus, e.g., Gaussian white noise received at gradiometer 110. The legs 70 rest on or are epoxied onto the bottom of the faraday cage 10. In the illustrated embodiment, the frames 66 and 68 and legs 70 are made of glass fiber epoxy. Other arrangements of transformers or coils may be used around the sample tray 50 without departing from the spirit and scope of the invention.

Referring to Figure 4, there is shown a cross-sectional view of the faraday cage and its contents, showing windings 62 of Helmholtz transformer 60 in relation to dewar 100 and faraday cage 10. Note also in Figure 4 the positioning of sample tray 50 and sample 200.

Referring again to Figure 1, an amplitude adjustable Gaussian white noise stimulus generator 80 is external to magnetic shielding cage 40, and is electrically connected to the Helmholtz transformer 60 through filter 90 by electrical cable 82. As will be discussed below, sources of magnetic-field stimulus injected into the sample during signal recording other than a Gaussian noise generator may be employed. It will therefore be recognized in the description that follows that the Gaussian generator is simply exemplary of a source of magnetic-field stimulus that is injected into the recording system during signal recording.

Referring to Figure 3, cable 82 is run through side opening 12, attenuation tube 24, and through cap 32 via hole 34. Cable 82 is a co-axial cable further comprising a twisted pair of copper conductors 84 surrounded by interior and exterior magnetic shielding 86 and 88, respectively. In other embodiments, the conductors can be any nonmagnetic electrically conductive material, such as silver or gold. The interior and exterior magnetic shielding 86 and 88 terminates at cap 32, leaving the twisted pair 84 to span the remaining distance from the end cap to the Helmholtz transformer 60 shown in Figure 1. The interior magnetic shielding 86 is electrically connected to Faraday cage 16 through cap 32, while the exterior magnetic shielding is electrically connected to the magnetically shielded cage 40 shown in Figure 1.

Referring to Figure 1, the Gaussian white noise stimulus generator 80 can generate a nearly flat frequency spectrum from zero to 100 kilohertz, and at a selected voltage amplitude, e.g., between .01 to 1.0 volts, that produces a selected calculated magnetic field at the same of between 0-1 G (Gauss), e.g., over increments of 25 mG. In the illustrated embodiment, the filter 90 filters out noise above 50 kilohertz, but other frequency ranges may be used without departing from the spirit and scope of the invention.

The Gaussian white noise stimulus may be replaced by other stimulus signal patterns. Examples of such patterns include scanning a range of sine wave frequencies, a square wave, time-series data containing defined non-linear structure, or the SQUID output itself. These signals may themselves be pulsed between off and on states to further modify the stimulus signal. The white noise naturally generated by the magnetic shields may also be used as the source of the stimulus signal. In one embodiment, the source of a magnetic-field stimulus is simply a adjustable-voltage DC source that is operated to supply a DC voltage (offset) to the magnetic-stimulus coils, as a selected voltage, e.g., .01 to 1.0 volts, that produces a calculated magnetic field at the sample between 0 and 1 G (gauss). In still another embodiment, the source of the magnetic-field stimulus is a frequency-sweep generator that is operator to produce successive, sweeps over a selected frequency of preferably at least 0 to 1 kH, and typically 0 to 10kHz or higher. The sweep time is preferably 1 to 10 seconds, and at a selected voltage level, e.g., between 0.01 to 1.0 volts, that produces a selected calculated magnetic field at the sample of between 0 and 1 G. Thus the sweep generator might be set to produce successive frequency sweep every five seconds, over a sweep frequency between 1 to 10Khz, and a selected voltage level.

While not intending to be bound by a particular mechanism or model of signal generation, it appears that the injected magnetic-field stimulus is acting to stimulate or amplify certain low-frequency events or modes in the sample, such that the recorded time-domain signal is composed of these events superimposed on the signal background. Where the injected magnetic-field stimulus is white noise, the mechanism of stimulus may involve stochastic resonance. Where the magnetic-field stimulus is a DC offset, the stimulus may function to stimulate a nuclear or electron resonance process, in which case the recorded signal would have NMR or ESR components. Where the magnetic-field stimulus is a sweep frequency generator, the stimulus may serve to excite those low-frequency events corresponding to the instantaneous frequencies seen by the sample.

Gaussian white noise stimulus generator 80 is also electrically connected to the other input of dual trace oscilloscope 160 through patch cord 164.

Referring to Figures 1, 2 and 3, a sample of the substance 200 to be measured is placed on the sample tray 50 and the sample tray is placed within the faraday cage 10. In the first embodiment, the Gaussian white noise stimulus generator 80 is used to inject Gaussian white noise stimulus through the Helmholtz transformer 60. The noise signal creates an induced voltage in the gradiometer 110. The induced voltage in the gradiometer 110 is then detected and amplified by the SQUID 120, the output from the SQUID is further amplified by the flux locked loop 140 and sent to the SQUID controller 150, and then sent to the dual trace oscilloscope 160. The dual trace oscilloscope 160 is also used to display the signal generated by Gaussian white noise stimulus generator 80.

The Gaussian white noise stimulus signal (or other magnetic-field stimulus) is adjusted by altering the output of the stimulus generator 80 and by rotating the Helmholtz transformer 60 around the sample 200, shown in Figure 2. Rotation of the Helmholtz transformer 60 about the axis of the hinged connection of frames 66 and 68 alters its phasing with respect to the gradiometer 110. Depending upon the desired phase alteration, the hinged connection of frames 66 and 68 permits windings 62 and 64 to remain parallel to one another while rotating approximately 30 to 40 degrees around sample tray 50. The hinged connection also permits windings 62 and 64 to rotate as much as approximately 60 degrees out of parallel, in order to alter signal phasing of the field generated by Helmholtz transformer 60 with respect to gradiometer 110. The typical adjustment of phase will include this out-of-parallel orientation, although the other orientation may be preferred in certain circumstances, to accommodate an irregularly shaped sample 200, for example. Stimulus is applied at a selected stimulus "condition," that is, selected voltage when applying white noise or a DC offset, and a selected sweep frequency range, a repeat period, and a voltage level for a sweep stimulus.

Embodiments of the present invention provide a method and apparatus for detecting extremely low-threshold molecular electromagnetic signals without external interference. They further provide for the output of those signals in a format readily usable by a wide variety of signal recording and processing equipment.

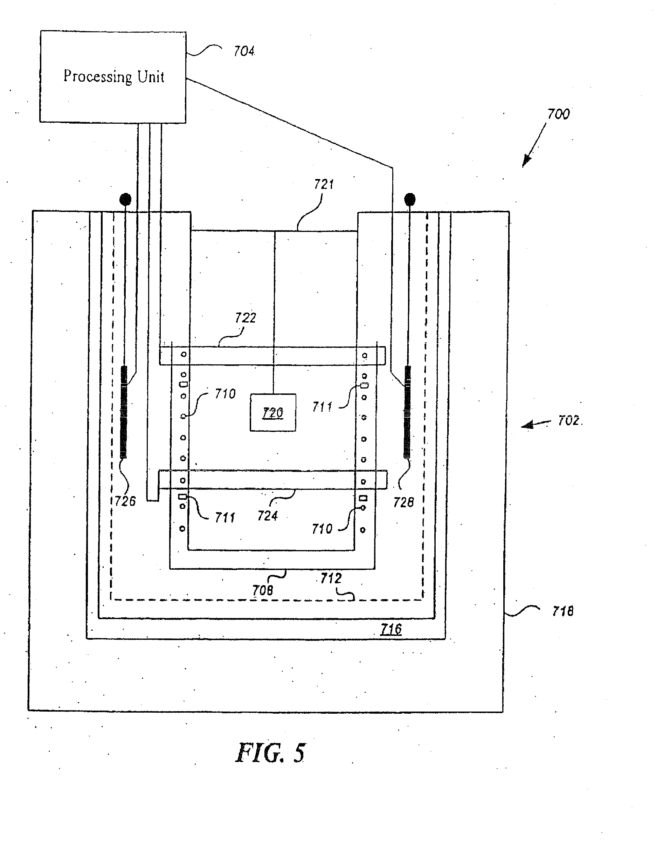

Referring now to Figure 5, an alternative embodiment to the molecular electromagnetic emission detection and processing system of the above figures is shown. A system 700 includes a detection unit 702 coupled to a processing unit 704. Although the processing unit 704 is shown external to the detection unit 702, at least a part of the processing unit can be located within the detection unit.

The detection unit 702, which is shown in a cross-sectional view in Figure 5, includes multiple components nested or concentric with each other. A sample chamber or faraday cage 706 is nested within a metal cage 708. Each of the sample chamber 706 and the metal cage 708 can be comprised of aluminum material. The sample chamber 706 can be maintained in a vacuum and may be temperature controlled to a preset temperature. The metal cage 708 is configured to function as a low pass filter.

Between the sample chamber 706 and the metal cage 708 and encircling the sample chamber 706 are a set of parallel heating coils or elements 710. One or more temperature sensor 711 is also located proximate to the heating elements 710 and the sample chamber 706. For example, four temperature sensors may be positioned at different locations around the exterior of the sample chamber 706. The heating elements 710 and the temperature sensor(s) 711 may be configured to maintain a certain temperature inside the sample chamber 706.

A shield 712 encircles the metal cage 708. The shield 712 is configured to provide additional magnetic field shielding or isolation for the sample chamber 706. The shield 712 can be comprised of lead or other magnetic shielding materials. The shield 712 is optional when sufficient shielding is provided by the sample chamber 706 and/or the metal cage 708.

Surrounding the shield 712 is a cryogen layer 716 with G10 insulation. The cryogen may be liquid helium. The cryogen layer 716 (also referred to as a cryogenic Dewar) is at an operating temperature of 4 degrees Kelvin. Surrounding the cryogen layer 716 is an outer shield 718. The outer shield 718 is comprised of nickel alloy and is configured to be a magnetic shield. The total amount of magnetic shielding provided by the detection unit 702 is approximately -100 dB, -100 dB, and -120 dB along the three orthogonal planes of a Cartesian coordinate system.

The various elements described above are electrically isolated from each other by air gaps or dielectric barriers (not shown). It should also be understood that the elements are not shown to scale relative to each other for ease of description.

A sample holder 720 can be manually or mechanically positioned within the sample chamber 706. The sample holder 720 may be lowered, raised, or removed from the top of the sample chamber 706. The sample holder 720 is comprised of a material that will not introduce Eddy currents and exhibits little or no inherent molecular rotation. As an example, the sample holder 720 can be comprised of high quality glass or Pyrex.

The detection unit 702 is configured to handle solid, liquid, or gas samples. Various sample holders may be utilized in the detection unit 702. For example, depending on the size of the sample, a larger sample holder may be utilized. As another example, when the sample is reactive to air, the sample holder can be configured to encapsulate or form an airtight seal around the sample. In still another example, when the sample is in a gaseous state, the sample can be introduced inside the sample chamber 706 without the sample holder 720. For such samples, the sample chamber 706 is held at a vacuum. A vacuum seal 721 at the top of the sample chamber 706 aids in maintaining a vacuum and/or accommodating the sample holder 720.

A sense coil 722 and a sense coil 724, also referred to as detection coils, are provided above and below the sample holder 720, respectively. The coil windings of the sense coils 722, 724 are configured to operate in the direct current (DC) to approximately 50 kilohertz, (kHz) range, with a center frequency of 25 kHz and a self-resonant frequency of 8.8 MHz. The sense coils 722, 724 are in the second derivative form and are configured to achieve approximately 100% coupling. In one embodiment, the coils 722, 724 are generally rectangular in shape and are held in place by G10 fasteners. The coils 722, 724 function as a second derivative gradiometer.

Helmholtz coils 726 and 728 may be vertically positioned between the shield 712 and the metal cage 708, as explained herein. Each of the coils 726 and 728 may be raised or lowered independently of each other. The coils 726 and 728, also referred to as magnetic-field stimulus generation coils, are at room or ambient temperature. The noise generated by the coils 726, 728 is approximately 0.10 Gauss.

The degree of coupling between the emissions from the sample and the coils 722, 724 may be changed by repositioning the sample holder 720 relative to the coils 722, 724, or by repositioning one or both of the coils 726, 728 relative to the sample holder 720.

The processing unit 704 is electrically coupled to the coils 722, 724, 726, and 728. The processing unit 704 specifies the magnetic-field stimulus, e.g., Gaussian white noise stimulus to be injected by the coils 726, 728 to the sample. The processing unit 104 also receives the induced voltage at the coils 722, 724 from the sample's electromagnetic emissions mixed with the injected magnetic-field stimulus.

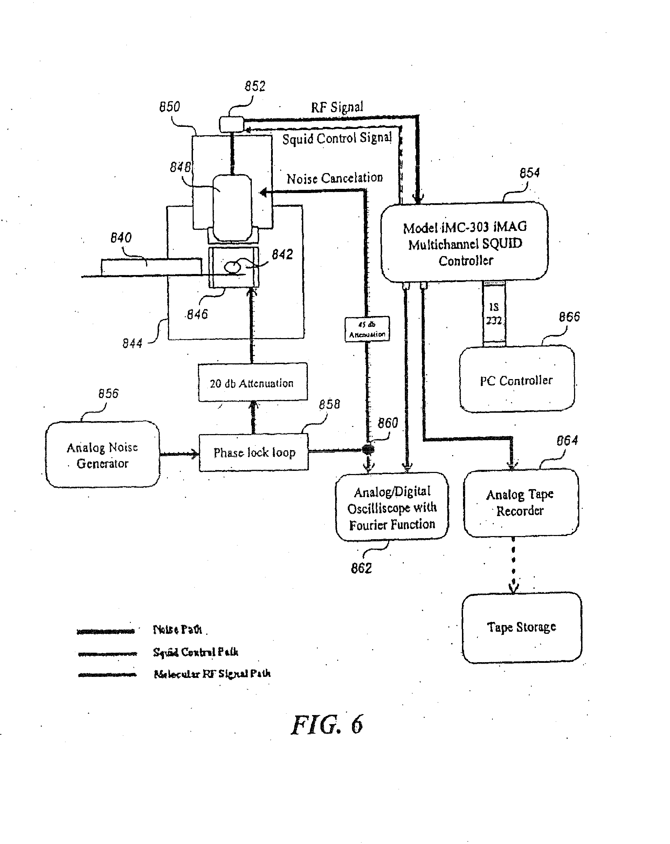

Referring to Figure 6, a processing unit employing aspects of the invention includes a sample tray 840 that permits a sample 842 to be inserted into, and removed from, a Faraday cage 844 and Helmholtz coil 746. A SQUID/gradiometer detector assembly 848 is positioned within a cryogenic dewar 850. A flux-locked loop 852 is coupled between the SQUID/gradiometer detector assembly 848 and a SQUID controller 854. The SQUID controller 854 may be a model iMC-303 iMAG multichannel controller provided by Tristan Technologies, Inc.

An analog Gaussian white noise stimulus generator 856 provides a noise signal (as noted above) to a phase lock loop 858. The x-axis output of the phase lock loop is provided to the Helmholtz coil 846, and may be attenuated, such as by 20 dB. The y-axis output of the phase lock loop is split by a signal splitter 860. One portion of the y-axis output is input to the noise cancellation coil at the SQUID, which has a separate input for the gradiometer. The other portion of the y-axis signal is input oscilloscope 862, such as an analog/digital oscilloscope having Fourier functions like the Tektronix TDS 3000b (e.g., model 3032b). That is, the x-axis output of the phase lock loop drives the Helmholtz coil, and the y-axis output, which is in inverted form, is split to input the SQUID and the oscilloscope. Thus, the phase lock loop functions as a signal inverter. The oscilloscope trace is used to monitor the analog magnetic-field stimulus signal. An analog tape recorder or recording device 864, coupled to the controller 854, records signals output from the device, and is preferably a wideband (e.g. 50 kHz) recorder. A PC controller 866 may be an MS Windows based PC interfacing with the controller 854 via, for example, an RS 232 port.

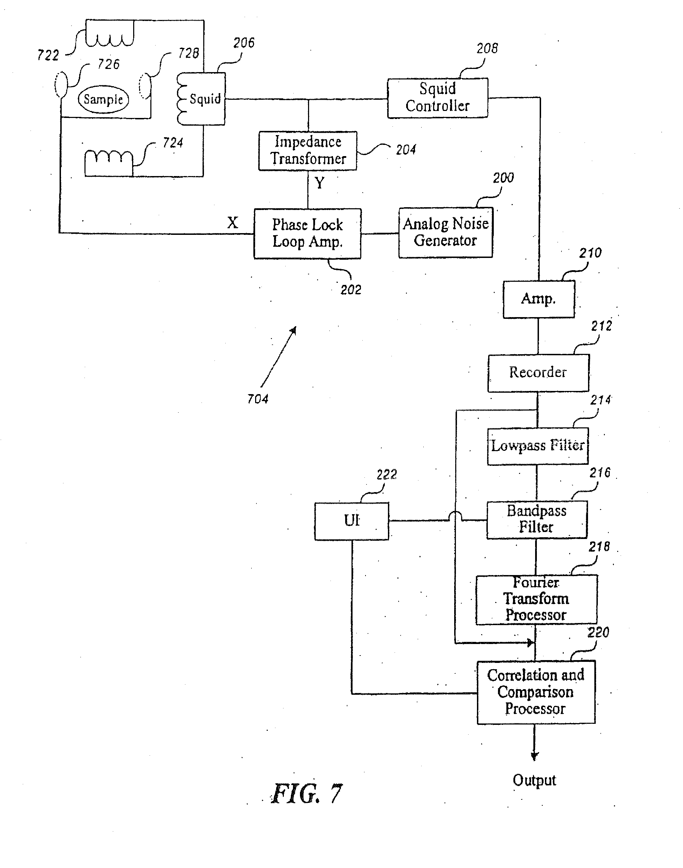

In Figure 7, a block diagram of another embodiment of the processing unit is shown. A dual phase lock-in amplifier 202 is configured to provide a first magnetic-field signal (e.g., "x" or noise stimulus signal) to the coils 726, 728 and a second magnetic-field signal (e.g., "y" or noise cancellation signal) to a noise cancellation coil of a superconducting quantum interference device (SQUID) 206. The amplifier 202 is configured to lock without an external reference and may be a Perkins Elmer model 7265 DSP lock-in amplifier. This amplifier works in a "virtual mode," where it locks to an initial reference frequency, and then removes the reference frequency to allow it to run freely and lock to "noise."

A magnetic-field stimulus generator, such as an analog Gaussian white noise stimulus generator 200 is electrically coupled to the amplifier 202. The generator 200 is configured to generate a selected magnetic-field stimulus, e.g., analog Gaussian white noise stimulus at the coils 726, 728 via the amplifier 202. As an example, the generator 200 may be a model 1380 manufactured by General Radio.

An impedance transformer 204 is electrically coupled between the SQUID 206 and the amplifier 202. The impedance transformer 204 is configured to provide impedance matching between the SQUID 206 and amplifier 202.

The SQUID 206 is a low temperature direct element SQUID. As an example, the SQUID 206 may be a model LSQ/20 LTS dC SQUID available form Tristan Technologies, Inc (San Diego, CA.) Alternatively, a high temperature or alternating current SQUID can be used. The coils 722, 724 (e.g., gradiometer) and the SQUID 206 (collectively referred to as the SQUID/gradiometer detector assembly) combined has a magnetic field measuring sensitivity of approximately 5 microTesla/$\sqrt{Hz}$. The induced voltage in the coils 722, 724 is detected and amplified by the SQUID 206. The output of the SQUID 206 is a voltage approximately in the range of 0.2-0.8 microVolts.

The output of the SQUID 206 is the input to a SQUID controller 208. The SQUID controller 208 is configured to control the operational state of the SQUID 206 and further condition the detected signal. As an example, the SQUID controller 208 may be an iMC-303 iMAG multi-channel SQUID controller manufactured by Tristan Technologies, Inc.

The output of the SQUID controller 208 is inputted to an amplifier 210. The amplifier 210 is configured to provide a gain in the range of 0-100 dB. A gain of approximately 20 dB is provided when noise cancellation node is turned on at the SQUID 206. A gain of approximately 50 dB is provided when the SQUID 206 is providing no noise cancellation.

The amplified signal is inputted to a recorder or storage device 212. The recorder 212 is configured to convert the analog amplified signal to a digital signal and store the digital signal. In one embodiment, the recorder 212 stores 8600 data points per Hz and can handle 2.46 Mbits/sec. As an example, the recorder 212 may be a Sony digital audiotape (DAT) recorder. Using a DAT recorder, the raw signals or data sets can be sent to a third party for display or specific processing as desired.

A lowpass filter 214 filters the digitized data set from the recorder 212. The lowpass filter 214 is an analog filter and may be a Butterworth filter. The cutoff frequency is at approximately 50 kHz.

A bandpass filter 216 next filters the filtered data sets. The bandpass filter 216 is configured to be a digital filter with a bandwidth between DC to 50 kHz. The bandpass filter 216 can be adjusted for different bandwidths.

The output of the bandpass filter 216 is the input to a Fourier transformer processor 218. The Fourier transform processor 218 is configured to convert the data set, which is in the time domain, to a data set in the frequency domain. The Fourier transform processor 218 performs a Fast Fourier Transform (FFT) type of transform.

The Fourier transformed data sets are the input to a correlation and comparison processor 220. The output of the recorder 212 is also an input to the processor 220. The processor 220 is configured to correlate the data set with previously recorded data sets, determine thresholds, and perform noise cancellation (when no noise cancellation is provided by the SQUID 206). The output of the processor 220 is a final data set representative of the spectrum of the sample's molecular low frequency electromagnetic emissions.

A user interface (UI) 222, such as a graphical user interface (GUI), may also be connected to at least the filter 216 and the processor 220 to specify signal processing parameters. The filter 216, processor 218, and the processor 220 can be implemented as hardware, software, or firmware. For example, the filter 216 and the processor 218 may be implemented in one or more semiconductor chips. The processor 220 may be software implemented in a computing device.

This amplifier works in a "virtual mode," where it locks to an initial reference frequency, and then removes the reference frequency to allow it to run freely and lock to "noise." The analog noise generator (which is produced by General Radio, a truly analog noise generator) requires 20 dB and 45-dB attenuation for the Helmholtz and noise cancellation coil, respectively.

The Helmholtz coil may have a sweet spot of about one cubic inch with a balance of 1/100th of a percent. In an alternative embodiments, the Helmholtz coil may move both vertically, rotationally (about the vertical axis), and from parallel to spread apart in a pie shape. In one embodiment, the SQUID, gradiometer, and driving transformer (controller) have values of 1.8, 1.5 and 0.3 micro-Henrys, respectively. The Helmholtz coil may have a sensitivity of 0.5 Gauss per amp at the sweet spot.

Approximately 10 to 15 microvolts may be needed for a stochastic response. By injecting Gaussian white noise stimulus, the system has raised the sensitivity of the SQUID device. The SQUID device had a sensitivity of about 5 femtotesla without the noise. This system has been able to improve the sensitivity by 25 to 35 dB by injecting noise and using this stochastic resonance response, which amounts to nearly a 1,500% increase.

After receiving and recording signals from the system, a computer, such as a mainframe computer, supercomputer or high-performance computer does both pre and post processing, such by employing the Autosignal software product by Systat Software of Richmond CA, for the pre-processing, while Flexpro software product does the post-processing. Flexpro is a data (statistical) analysis software supplied by Dewetron, Inc. The following equations or options may be used in the Autosignal and Flexpro products.

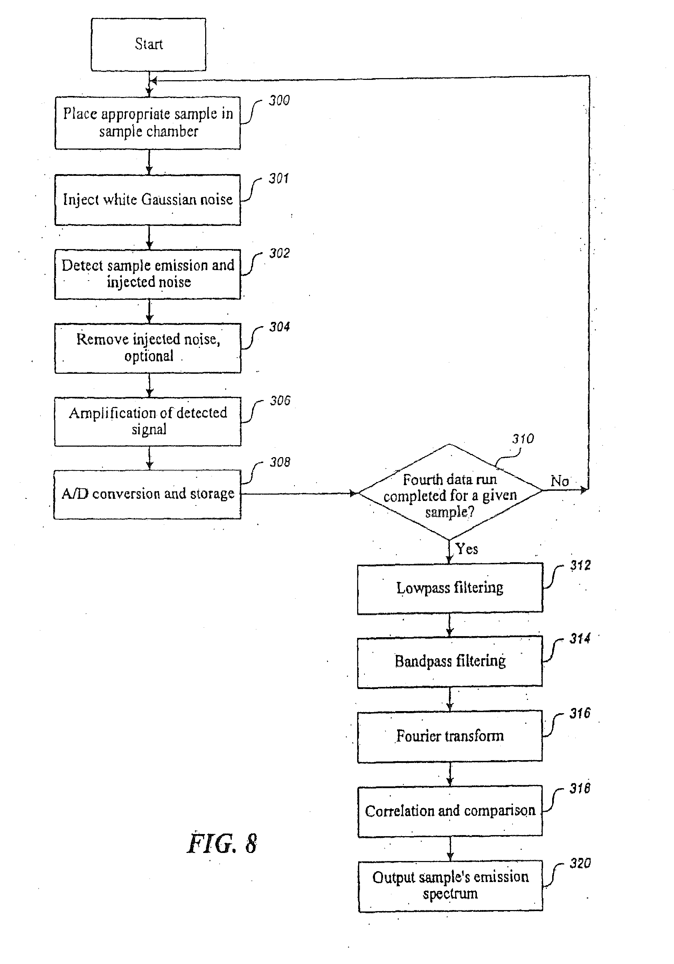

A flow diagram of the signal detection and processing performed by the system 100 is shown in Figure 8. When a sample is of interest, at least four signal detections or data runs are performed: a first data run at a time $t_1$ without the sample, a second data run at a time $t_2$ with the sample, a third data run at a time $t_3$ with the sample, and a fourth data run at a time $t_4$ without the sample. Performing and collecting data sets from more than one data run increases accuracy of the final (e.g., correlated) data set. In the four data runs, the parameters and conditions of the system 100 are held constant (e.g., temperature, amount of amplification, position of the coils, the Gaussian white noise stimulus signal, etc.).

At block 300, the appropriate sample (or if it's a first or fourth data run, no sample), is placed in the system 100. A given sample, without injected Gaussian white noise stimulus, emits electromagnetic emissions in the DC-50 kHz range at an amplitude equal to or less than approximately 0.001 microTesla. To capture such low emissions, Gaussian white noise stimulus is injected at block 301.

At block 302, the coils 722, 724 detect the induced voltage representative of the sample's emission and the injected Gaussian white noise stimulus. The induced voltage comprises a continuous stream of voltage values (amplitude and phase) as a function of time for the duration of a data run. A data run can be 2-20 minutes in length and hence, the data set corresponding to the data run comprises 2-20 minutes of voltage values as a function of time.

At block 304, the injected Gaussian white noise stimulus is cancelled as the induced voltage is being detected. This block is omitted when the noise cancellation feature of the SQUID 206 is turned off.

At block 306, the voltage values of the data set are amplified by 20-50 dB, depending on whether noise cancellation occurred at the block 304. And at- block 308, the amplified data set undergoes analog to digital (A/D) conversion and is stored in the recorder 212. A digitized data set can comprise millions of rows of data.

After the acquired data set is stored, at a block 310 a check is performed to see whether at least four data runs for the sample have occurred (e.g., have acquired at least four data sets). If four data sets for a given sample have been obtained, then lowpass filtering occurs at block 312. Otherwise, the next data run is initiated (return to the block 300).

After lowpass filtering (block 312) and bandpass filtering (at a block 314) the digitized data sets, the data sets are converted to the frequency domain at a Fourier transform block 316.

Next, at block 318, like data sets are correlated with each other at each data point. For example, the first data set corresponding to the first data run (e.g., a baseline or ambient noise data run) and the fourth data set corresponding to the fourth data run (e.g., another noise data run) are correlated to each other. If the amplitude value of the first data set at a given frequency is the same as the amplitude value of the fourth data set at that given frequency, then the correlation value or number for that given frequency would be 1.0. Alternatively, the range of correlation values may be set at between 0-100. Such correlation or comparison also occurs for the second and third data runs (e.g., the sample data runs). Because the acquired data sets are stored, they can be accessed at a later time as the remaining data runs are completed.

Predetermined threshold levels are applied to each correlated data set to eliminate statistically irrelevant correlation values. A variety of threshold values may be used, depending on the length of the data runs (the longer the data runs, greater the accuracy of the acquired data) and the likely similarity of the sample's actual emission spectrum to other types of samples. In addition to the threshold levels, the correlations are averaged. Use of thresholds and averaging correlation results in the injected Gaussian white noise stimulus component becoming very small in the resulting correlated data set.

Once the two sample data sets have been refined to a correlated sample data set and the two noise data sets have been refined to a correlated noise data set, the correlated noise data set is subtracted from the correlated sample data set. The resulting data set is the final data set (e.g., a data set representative of the emission spectrum of the sample) (block 320).

Since there can be 8600 data points per Hz and the final data set can have data points for a frequency range of DC-50 kHz, the final data set can comprise several hundred million rows of data. Each row of data can include the frequency, amplitude, phase, and a correlation value.

III. Method of identifying candidate optimal time-domain signals

The signals produced in accordance with the methods described above may be further selected for optimal effector activity, when used to transduce an in vitro or mammalian system. According to one aspect of the invention, it has been discovered that sample-dependent signal features in a low-frequency time-domain signal obtained for a given sample can be optimized by recording time-domain signals for sample over a range of magnetic-field stimulus conditions, e.g., different voltage levels for Gaussian white noise stimulus amplitudes and DC offsets. The recorded signals are then processed to reveal signal features, and one or more time domain signals having an optimal signal-analysis score, as detailed below, are selected. The selection of optimized or near-optimized time-domain signals is useful because it has been found, also in accordance with the invention, that transducing an *in vitro* or biological system with an optimized time-domain signal gives a stronger and more predictable response than with a non-optimized time-domain signal. Viewed another way, selecting an optimized (or near-optimized) time-domain signal is useful in achieving reliable, detectable sample effects when a target system is transduced by the sample signal.

In general, the range of injected white noise, DC offset, and sweep amplitude voltages applied to the sample are such as to produce a calculated magnetic field at the sample container of between 0 to 1 G (Gauss), or alternatively, the injected noise stimulus is preferably between about 30 to 35 decibels above the molecular electromagnetic emissions sought to be detected, e.g., in the range 70-80-dbm. The number of samples that are recorded, that is, the number of noise-level intervals over which time-domain signals are recorded may vary from 10-100 or more, typically, and in any case, at sufficiently small intervals so that a good optimum signal can be identified. For example, the power gain of the noise generator level can be varied over 50 20 mV intervals. As will be seen below, when the signal-analysis scores for the signals are plotted against level of injected noise stimulus, the plot shows a peak extending over several different noise levels when the noise-level increments are suitable small.

Alternatively, stimulus signals other than Gaussian white noise can be used for optimization of the recorded time-domain signal. Examples of such signals include scanning a range of sine wave frequencies, a square wave, time-series data containing defined non-linear structure, or the SQUID output itself. These signals may themselves be pulsed between off and on states to further modify the stimulus signal. The white noise naturally generated by the magnetic shields may also be used as the source of the stimulus signal.

The present invention contemplates five different methods for calculating signal-analysis scores for the recorded time-domain signals. These are (A) a histogram bin method, (B) generating an FFT of autocorrelated signals, (C) averaging of FFTs, (D) use of a cross-correlation threshold, and (E) phase-space comparison. Each of these is detailed below

Although not specifically described, it will be appreciated that each method may be carried out in a manual mode, where the user evaluates the spectra on which a signal-analysis score is based, makes the noise stimulus level adjustment for the next recording, and determines when a peak score is reached, or it may be carried out in an automated or semi-automated mode, in which the continuous incrementing of noise stimulus level and/or the evaluation of signal-analysis score, is performed by a computer-driven program.

A. Histogram Method of Generating Spectral Information

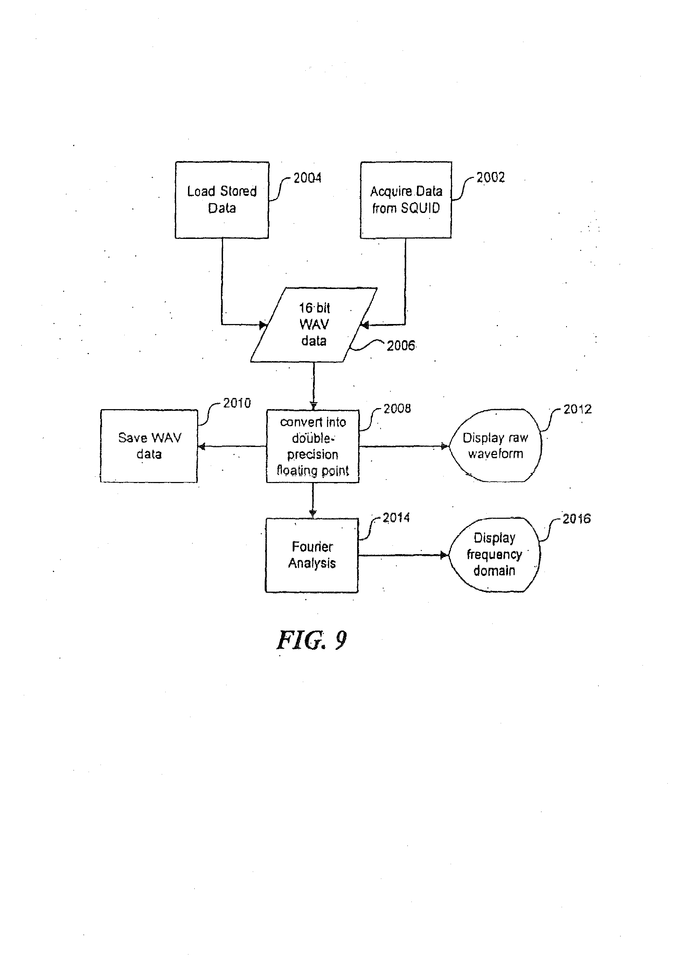

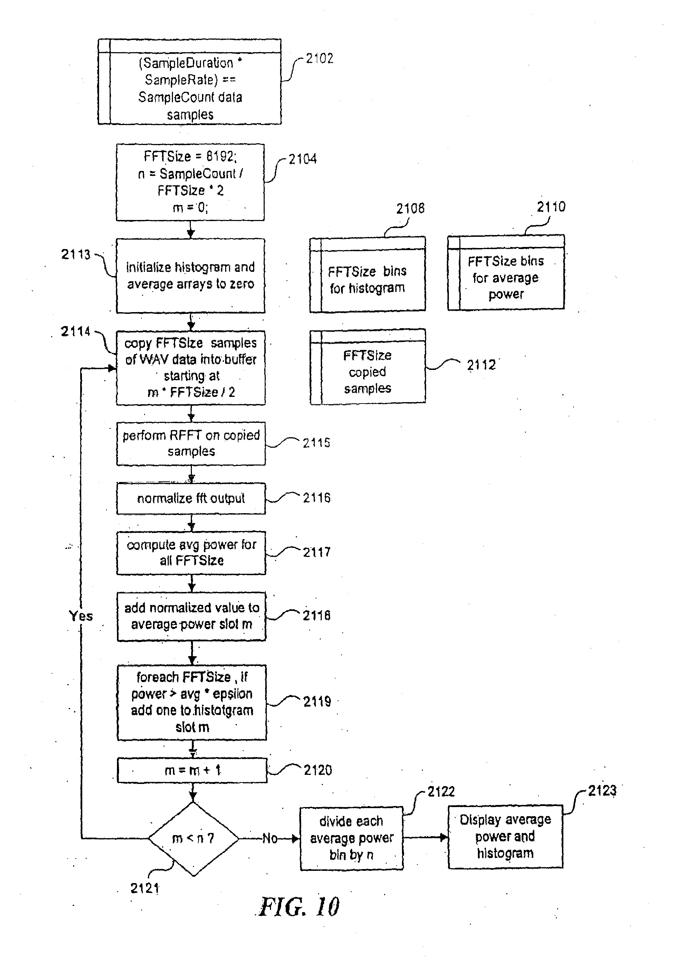

Figure 9 is a high level data flow diagram in the histogram method for generating spectral information. Data acquired from the SQUID (box 2002) or stored data (box 2004) is saved as 16 bit WAV data (box 2006), and converted into double-precision floating point data (box 2008). The converted data may be saved (box 2010) or displayed as a raw waveform (box 2012). The converted data is then passed to the algorithm described below with respect to Figure 10, and indicated by the box 2014 labeled Fourier Analysis. The histogram can be displayed at 2016.

With reference to Figure 10, the general flow of the histogram algorithm is to take a discrete sampled time-domain signal and use Fourier analysis to convert it to a frequency domain spectrum for further analysis. The time-domain signals are acquired from an ADC (analog/digital converter) and stored in the buffer indicated at 2102. This sample is *SampleDuration* seconds long, and is sampled at *SampleRate* samples per second, thus providing *SampleCount* (*SampleDuration * SampleRate)* samples. The *FrequencyRange* that can be recovered from the signal is defined as half the *SampleRate,* as defined by Nyquist. Thus, if a time-series signal is sampled at 10,000 samples per second, the *FrequencyRange* will be 0 Hz to 5 kHz. One Fourier algorithm that may be used is a Radix 2 Real Fast Fourier Transform (RFFT), which has a selectable frequency domain resolution (*FFTSize*) of powers of two up to $2^{16}$. An *FFTSize* of 8192 is selected, to provide provides enough resolution to have at least one spectrum bin per Hertz as long as the *FrequencyRange* stays at or below 8 kHz. The *SampleDuration* should be long enough such that *SampleCount >*(**2**\*) *FFTSize \* 10* to ensure reliable results.

Since this FFT can only act on FFTSize samples at a time, the program must perform the FFT on the samples sequentially and average the results together to get the final spectrum. If one chooses to skip *FFTSize* samples for each FFT, a statistical error of 1 / FFTSize ^ 0.5 is introduced. If, however, one chooses to overlap the FFT input by half the *FFTSize,* this error is reduced to 1 / (0.81\*2\* *FFTSize*) ^ 0.5. This reduces the error from 0.0110485435 to 0.0086805556. Additional information about errors and correlation analyses in general, consult Bendat & Piersol, "Engineering Applications of Correlation and Spectral Analysis", 1993.

Prior to performing the FFT on a given window, a data tapering filter may be applied to avoid spectral leakage due to sampling aliasing. This filter can be chosen from among Rectangular (no filter), Hamming, Hanning, Bartlett, Blackman and Blackman/Harris, as examples.

In an exemplary method, and as shown in box 2104, we have chosen 8192 for the variable FFTSize, which will be the number of time-domain samples we operate on at a time, as well as the number of discrete frequencies output by the FFT. Note that FFTSize =8192 is the resolution, or number of bins in the range which is dictated by the sampling rate. The variable n, which dictates how many discrete RFFT's (Real FFT's) performed, is set by dividing the SampleCount by FFTSize *2, the number of FFT bins. In order for the algorithm to generate sensible results, this number n should be at least 10 to 20 (although other valves are possible), where more may be preferred to pick up weaker signals. This implies that for a given SampleRate and FFTSize, the SampleDuration must be long enough. A counter m, which counts from 0 to n, is initialized to zero, also as shown in box 2104.

The program first establishes three buffers: buffer 2108 for FFTSize histogram bins, that will accumulate counts at each bin frequency; buffer 2110 for average power at each bin frequency, and a buffer 2112 containing the FFTSize copied samples for each m.

The program initializes the histograms and arrays (box 2113) and copies FFTSize samples of the wave data into buffer 2112, at 2114, and performs an RFFT on the wave data (box 2115). The FFT is normalized so that the highest amplitude is 1 (box 2116) and the average power for all FFTSize bins is determined from the normalized signal (box 2117). For each bin frequency, the normalized value from the FFT at that frequency is added to each bin in buffer 2108 (box 2118).

In box 2119 the program then looks at the power at each bin frequency, relative to the average power calculated from above. If the power is within a certain factor epsilon. (between 0 and 1) of the average power, then it is counted and the corresponding bin is incremented in the histogram buffer at 16. Otherwise it is discarded.

Note that the average power it is comparing to is for this FFT instance only. An enhanced, albeit slower algorithm might take two passes through the data and compute the average over all time before setting histogram levels. The comparison to epsilon helps to represent a power value that is significant enough for a frequency bin. Or in broader terms, the equation employing epsilon helps answer the question, "is there a signal at this frequency at this time?" If the answer is yes, it could due be one of two things: (1) stationary noise which is landing in this bin just this one time, or (2) a real low level periodic signal which will occur nearly every time. Thus, the histogram counts will weed out the noise hits, and enhance the low level signal hits. So, the averaging and epsilon factor allow one to select the smallest power level considered significant.

Counter m is incremented at box 2120, and the above process is repeated for each n set of WAV data until m is equal to n (box 2121). At each cycle, the average power for each bin is added to the associated bin at 2118, and each histogram bin is incremented by one when the power amplitude condition at 2114 is met.

When all n cycles of data have been considered, the average power in each bin is determined by dividing the total accumulated average power in each bin by n, the total number of cycles (box 2122) and the results displayed (box 2123). Except where structured noise exists, e.g., DC = 0 or at multiples of 60 Hz, the average power in each bin will be some relatively low number.

The relevant settings in this method are noise stimulus gain and the value of epsilon. This value determines a power value that will be used to distinguish an event over average value. At a value of 1, no events will be detected, since power will never be greater than average power. As epsilon approaches zero, virtually every value will be placed in a bin. Between 0 and 1, and typically at a value that gives a number of bin counts between about 20-50% of total bin counts for structured noise, epsilon will have a maximum "spectral character," meaning the stochastic resonance events will be most highly favored over pure noise.

Therefore, one can systematically increase the power gain on the magnetic-field stimulus input, e.g., in 50 mV increments between 0 and 1 V, and at each power setting, adjust epsilon until a histogram having well defined peaks is observed. Where, for example, the sample being processed represents a 20 second time interval, total processing time for each different power and epsilon will be about 25 seconds. When a well-defined signal is observed, either the power setting or epsilon or both can be refined until an optimal histogram, meaning one with the largest number of identifiable peaks, is produced.

Under this algorithm, numerous bins may be filled and associated histogram rendered for low frequencies due to the general occurrence of noise (such as environmental noise) at the low frequencies. Thus, the system may simply ignore bins below a given frequency (e.g., below 1 kHz), but still render sufficient bin values at higher frequencies to determine unique signal signatures between samples.

Alternatively, since a purpose of the epsilon variable is to accommodate different average power levels determined in each cycle, the program could itself automatically adjust epsilon using a predefined function relating average power level to an optimal value of epsilon.

Similarly, the program could compare peak heights at each power setting, and automatically adjust the noise stimulus power setting until optimal peak heights or character is observed in the histograms.

Although the value of epsilon may be a fixed value for all frequencies, it is also contemplated to employ a frequency-dependent value for epsilon, to adjust for the higher value average energies that may be observed at low frequencies, e.g., DC to 1,000. A frequency-dependent epsilon factor could be determined, for example, by averaging a large number of low-frequency FFT regions, and determining a value of epsilon that "adjusts" average values to values comparable to those observed at higher frequencies.

B. <u>FFT of autocorrelated signals</u>

In a second general method for determining signal-analysis scores, time-domain signals recorded at a selected noise stimulus are autocorrelated, and a fast Fourier transform (FFT) of the autocorrelated signal is used to generate a signal-analysis plot, that is, a plot of the signal in the frequency domain. The FFTs are then used to score the number of spectral signals above an average noise level over a selected frequency range, e.g., DC to 1 kHz or DC to 8 kHz.

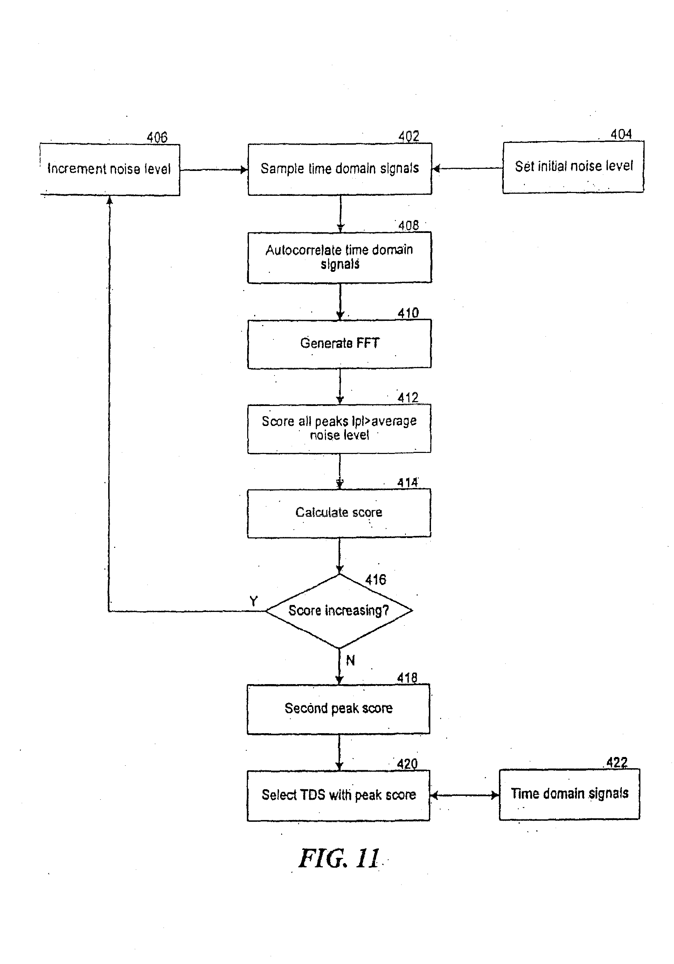

Figure 11 is a flow diagram of steps carried out in scoring recorded time-domain signals according to this second embodiment. Time-domain signals are sampled, digitized, and filtered as above (box 402), with the gain on the magnetic-field stimulus level set to an initial level, as at 404. A typical time domain signal for a sample compound 402 is autocorrelated, at 408, using a standard autocorrelation algorithm, and the FFT of the autocorrelated function is generated, at 410, using a standard FFT algorithm.

An FFT plot is scored, at 412, by counting the number of spectral peaks that are statistically greater than the average noise observed in the autocorrelated FFT and the score is calculated at 414. This process is repeated, through steps 416 and 406, until a peak score is recorded, that is, until the score for a given signal begins to decline with increasing noise stimulus gain. The peak score is recorded, at 418, and the program or user selects, from the file of time-domain signals at 422, the signal corresponding to the peak score (box 420).

As above, this embodiment may be carried out in a manual mode, where the user manually adjusts the noise stimulus setting in increments, analyzes (counts peaks) from the FFT spectral plots by hand, and uses the peak k score to identify one or more optimal time-domain signals. Alternatively, one or more aspects of the steps can be automated.

C. <u>Averaged FFTs</u>

In another embodiment for determining signal-analysis scores, an FFT of many, e.g., 10-20 time domain signals at each noise stimulus gain are averaged to produce a spectral-peaks plot, and scores are calculated as above.

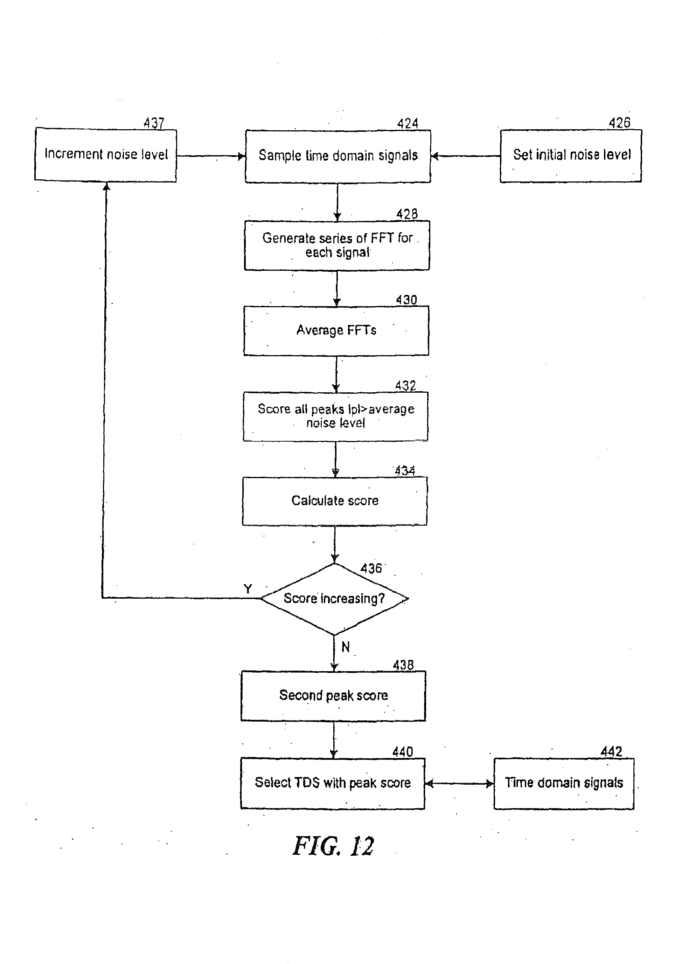

Figure 12 is a flow diagram of steps carried out in scoring recorded time-domain signals according to this third embodiment. Time-domain signals are sampled, digitized, and filtered as above (box 424), with the gain on the magnetic-field stimulus level set to an initial level, as at 426. The program then generates a series of FFTs for the time domain signal(s) at each noise stimulus gain, at 428, and these plots are averaged at 430. Using the averaged FFT plot, scoring is done by counting the number of spectral peaks that are statistically greater than the average noise observed in the averaged FFT, as at 432, 434. This process is repeated, through the logic of 436 and 437, until a peak score is recorded, that is, until the score for a given signals begins to decline with increasing magnetic-field stimulus gain. The peak score is recorded, at 438, and the program or user selects, from the file of time-domain signals at 442, the signal corresponding to the peak score (box 440).

As above, this method may be carried out in a manual, semi-automated, or fully automated mode.

D. <u>Cross-Correlation Threshold</u>

In another embodiment for determining signal-analysis scores, a cross-correlation algorithm is used in conjunction with a threshold. First, the response time-series data is offset such that its mean is zero, by calculating its mean value and subtracting that value from the whole of the data. Then, a block of data of duration Tau is extracted from near the beginning of this time-series data, and a cross-correlation performed with the remainder of the dataset. The algorithm for the cross-correlation is well-known. The cross-correlation output is used to calculate a standard deviation value. The algorithm for calculating the standard deviation is well-known. This standard deviation is then multiplied by a factor called Alpha, which is typically 2.0, to generate a threshold value. The cross-correlation output is then compared against this threshold value, and the number of times the cross-correlation output exceeds the threshold value is counted. The count value is the score for that response time-series data.

This method of calculating the score for response times-series data provides a measure of how often a data pattern contained within the Tau data block is repeated in the remainder of the data, and thus constitutes a measure of how much data pattern is being produced by the sample.

The score can be calculated for response time-series by data for a range of Tau durations, to ensure appropriate capture of data patterns produced by the sample.

Score values can be calculated for a sample under varying conditions, such as varying stimulus Gaussian white noise amplitude or offset. Comparison of the resulting set of score values enables identification of the sample conditions that produce the strongest data patterns from the sample. Those conditions can then be used for acquiring data for use in effecting chemical or biological systems.

In one embodiment, the system extracts time series data from a WAV-format file, representing the SQUID data recorded (for typically 60 s) from a MIDS unit. A block of data of duration *Tau* (typically 5 to 20 ms) is taken from near the beginning of the time series, and a cross-correlation is performed with the remainder of the data, yielding a cross-correlation dataset.

**CrossCorrelation Details**

The cross correlation Rxy(t) of the signals x(t) and y(t) is defined as

$$R_{xy}(t) = x(t) \otimes y(t) = \int_{-\infty}^{\infty} x(\tau)y(t+\tau)d\tau$$

where the symbol $\otimes$ denotes correlation.

The discrete implementation of the CrossCorrelation VI is as follows. Let h represent a sequence whose indexing can be negative, let n be the number of elements in the input sequence X, let m be the number of elements in the sequence Y, and assume that the indexed elements of X and Y that lie outside their range are equal to zero,

$$x_j = 0, j < 0 \text{ or } j \geq n$$

and

$$y_j = 0, j < 0 \text{ or } j \geq m.$$

Then the CrossCorrelation VI obtains the elements of h using

$$h_j = \sum_{k=0}^{n-1} x_k y_{j+k}$$

for j=-(n-1),-(n-2), ..,-2, -1, 0, 1, 2, ..., m-1

The elements of the output sequence **Rxy** are related to the elements in the sequence h by

$$Rxy_i = h_{i-(n-1)}$$

for i = 0,1,2, ..., size-1, size = n+m-1,
where size is the number of elements in the output sequence **Rxy**.

Then, the mean of this cross-correlation dataset is calculated, and multiplied by a factor Alpha (typically 1.1), to create a threshold value. The total number of times that the cross-correlation dataset crosses the threshold value is counted, and this count is output as the *Score* value.

The Score value is essentially a measure of how spiky the cross-correlation dataset is, and thus is a measure of how often a pattern (of duration *Tau)* in the initial data block is repeated in the subsequent data.

Since such a repetitive pattern may or may not exist in the initial data block, the position and size of the initial data block are varied to determine the statistical significance of resultant *Score* values.

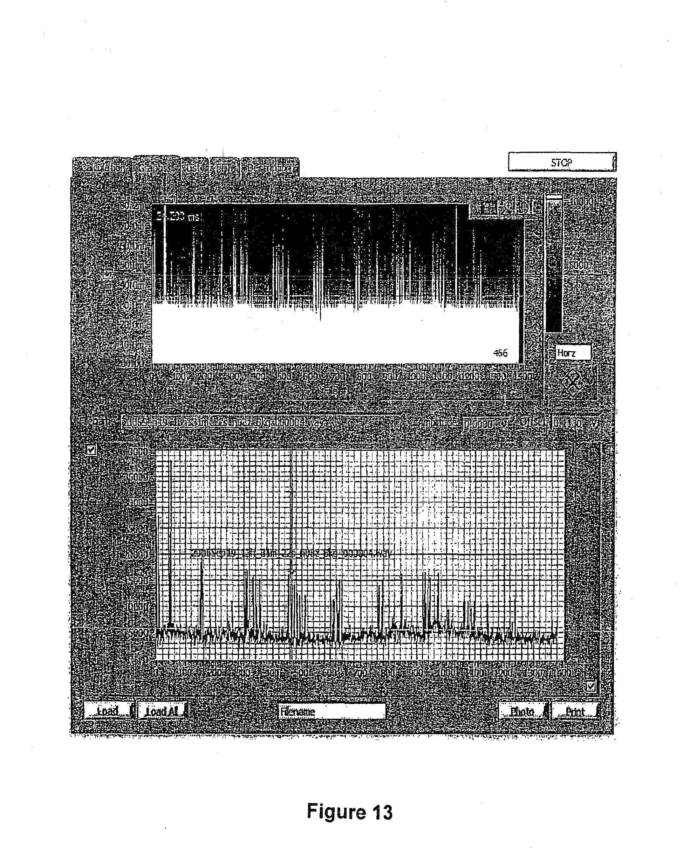

An example Score result is shown in Figure 13. The upper graph shows *File #* on the X -axis, *Tau* on the Y-axis, and *Score* on the Z-axis. The *File #* corresponds to a sequence of files accumulated with varying acquisition parameters; in this example, the MIDS stimulus offset is repeatedly incremented from +100 mV to +250 mV nine times. The *Tau* refers to the duration of the initial data block, which is incremented to ensure capture of any data patterns. The *Score* at each X,Y coordinate in indicated by a color map; in this example, a score of 0 is black, 5000 is blue, and 10000 is white, with intermediate values indicated by intermediate colors.

The user can manually move the red cursor around to slice through the intensity graph either horizontally or vertically; in this example, it is sliced horizontally, and the data across the slice is presented in the lower linear graph.

The lower linear graph indicates how the Score value changes. In this example, it shows how the *Score* typically is higher for files acquired with MIDS offsets in the range of +105 mV to +130 mV, a motif that repeats itself nine times in synchronization with the repetition of the offset incrementation. This indicates a data pattern (of unknown structure) present for the +105 mV to +130 mV offsets that is not present for the remaining offsets.

E. Phase-Space Comparison

in another embodiment for determining signal-analysis scores, a phase-space is computed for the response time-series data, and this phase-space correlated with the phase-space from another time-series data. First, the response time-series data is used to compute Average Mutual Information. The algorithm for Average Mutual information is well-known. The first minimum corresponds to an optimal Tau value. This Tau value is then used to compute an N-dimensional phase space using Takens' Theorem. The algorithm for Takens' Theorem is well-known.

The resulting phase-space structure is then compared with the phase-space structure from another time-series. The comparison would typically be between data acquired with sample present and sample absent, or between sample present and solvent-only present.

The comparison is performed by comparing the phase-space densities throughout the phase-space region. This is computed by calculating the weighted average of the absolute value of a quotient formed by dividing the natural logarithm of the probability array S by the probability array R. The probability arrays S and R are formed by binning the Sample phase space and the Reference phase space into a finite number of bins, then normalizing to 1. The output of the comparison is the score value.

Score values can be calculated for a sample under varying conditions, such as varying Gaussian white noise stimulus amplitude or offset. Comparison of the resulting set of score values enables identification of the sample conditions that produce the strongest data patterns from the sample. Those conditions can then be used for acquiring data for use in effecting chemical or biological systems. First, the incoming time series data ("Voltage Array in") is scaled so that its values are in the range of zero up to "Phase Space Size", which is typically 1000. Then, these values are discretized to integers. These integers are then used as indices of a two-dimensional array, the Phase Space, which is typically 1000x1000. Pairs of integers that are separated by a duration "Tau" are used to specify an X,Y location in the Phase Space, and the value at that location is incremented by a value of 1. All possible such pairs of integers are used by sliding along the time series data, which generates a net pattern in the Phase Space.

To compare two different Phase Spaces, such as from a sample and a reference, the two Phase Spaces (Sample Array and Reference Array) are normalized by dividing by their sums, to yield Ps(xyz) and Pr(xyz), respectively. Each element within the arrays is then used to compute a net difference: Difference = (sum ((sqrt(Ps(xyz)*Pr(xyz)))*(abs(in(Ps(xyz)/Pr(xyz))))))/(sum(sqrt(Ps(xyz)*Pr(xyz))))

The difference represents a Score value for the specific data acquisition conditions, such as a given stimulus noise amplitude and offset. Applying this Difference calculation to a set of data spanning a range of amplitudes and offsets thus provides a set of Score values. The highest Score value indicates where the sample has had the greatest non-linear influence on the data, and thus suggests it's effectiveness in biological transduction.

Of the five scoring algorithms, the preference is for (i) the FFT autocorrelation method (Algorithm B), (ii) the phase-space comparison (Algorithm E), or (iii) the histogram method (Algorithm A).

IV. Transduction apparatus and protocols

This section describes equipment and methodology for transducing a sample with signals generated and selected according to the methods described in Sections I and II above. The signals employed in these experiments, which are optimized time-domain signals formed in accordance with the method described above demonstrate the ability of signals in accordance with the invention to produce a compound-specific response in various in vitro or mammalian systems.

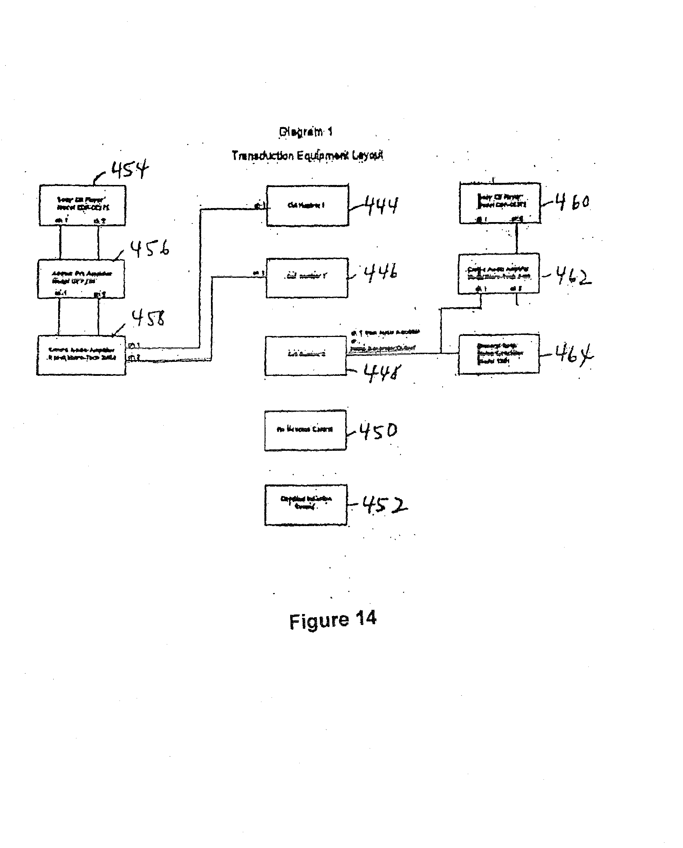

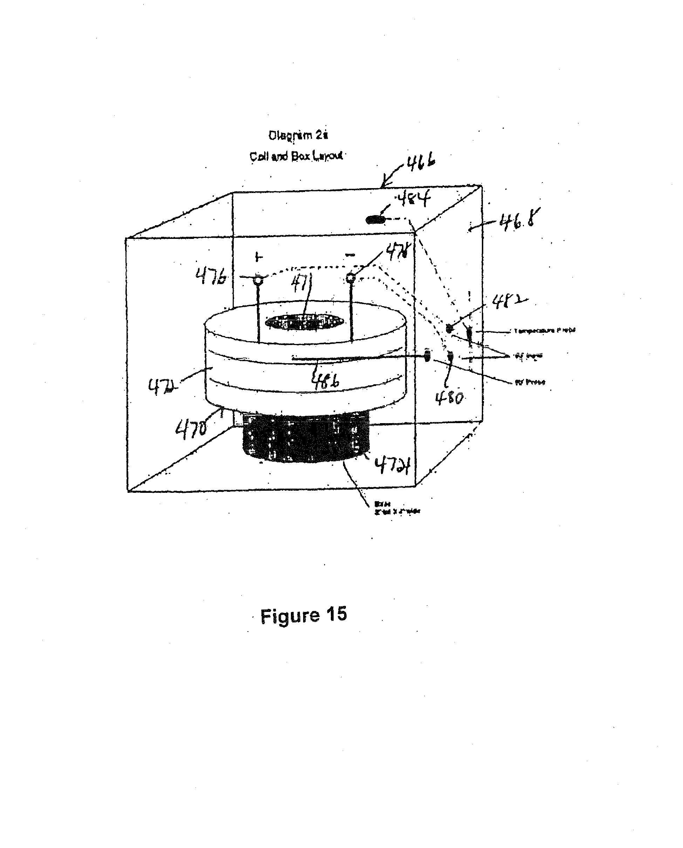

Figure 14 shows the layout of equipment for transducing a sample with an agent-specific signal, in accordance with the invention. The particular layout accommodates five different samples, including three samples 444, 446, and 448 which are held within transduction coils, and exposed to electromagnetic signals, a sample 450 that serves as a control, and a sample 452 that serves as a chemical-induction control. The system of Figure 15 may be used for experimentation; if used for treating a patient, then some elements may be omitted, such as 448, 450, 452, etc.

Transduction by an agent-specific signal is carried out by "playing" the optimized agent-specific signal to the sample, using, where the signal is recorded on a CD, and is played on a CD recorded 454 through a preamplifier 456 and an audio amplifier 458. This signal is supplied to the electromagnetic coils 444 and 446 through separate channels, as shown. In one embodiment, a Sony Model CDP CE375 CD Player is used. Channel 1 of the Player is connected to CD input 1 of Adcom Pre Amplifier Model GFP 750. Channel 2 is connected to CD input 2 of Adcom Pre Amplifier Model GFP 750. CD's are recorded to play identical signals from each channel. Alternatively, CD's may be recorded to play different signals from each channel. The coil in sample 448 is used primarily to produce a Gaussian white noise field as a control for experiments. For example, a GR analog noise generator provides a Gaussian white noise source for this coil. Alternatively, this coil can be used to play any pre recorded transduction signal via a second Crown amplifier.

Figure 15 shows sample transduction equipment 466 such as represented by any of samples 444, 446, and 448 in Figure 14. The equipment includes a chamber 468 housing an electromagnet 470, and various probes for monitoring conditions within the chamber, e.g., temperature. The electromagnet sits on a base 474, and includes, conventionally a toroidal ferromagnetic core and wire windings.

The electromagnet may have one or more windings for the purpose of controlling its magnetic field magnitude, gradient, and orientation in the region where the sample is placed.

In one embodiment of the transduction equipment, the coils are engineered and manufactured by American Magnetics to provide uniform performance between coils. Each coil consists of 416 turns of #8 gauge (awg) square copper magnet wire, enamel coated, with about a diameter 2" air core. Each coil can produce approximately 1500 Gauss in the center at 10 Volts RMS at 10 Amps RMS at 11 Hertz without exceeding a 15 degree Celsius rise in temperature.

In a second embodiment of the transduction equipment, suitable for use where the transducing component is a low-field NMR signal, or contains NMR components, a pair of coils may be axially separated by approximately the same distance as the diameter, forming a Helmholtz configuration. Electric current circulates in the same direction in both coils. This configuration optimizes the magnetic field uniformity in the vicinity of the center of the pair.

In a third embodiment of the transduction equipment, also suitable for use where the transducing component is a low-field NMR signal, or contains NMR components, two pairs of Helmholtz coils are wound on top of each other, where one pair has electric current circulating in the same direction in both coils, and the other pair has electric current circulating in opposite directions. This configuration produces a controlled magnetic field gradient, as well as a controlled magnetic field magnitude.

In another general embodiment of the transduction equipment, several Helmholtz coil pairs may be constructed to be orthogonal to one another. This configuration would allow considerable flexibility in controlling the structure of the magnetic field applied to a sample. For example, a static magnetic field could be applied along one axis, and a varying magnetic field applied along another axis. Such a configuration would be useful for applying NMR-type signals to biological systems. A static field of 7 microTesla would be generated by a constant current through the first coil, and a varying magnetic field of lesser amplitude would be generated by a varying current through the second coil. The varying current would be generated by a set of sine waves added together, where the sine wave have frequencies corresponding to the calculated NMR spectrum at 7 microTesla.

The transduction equipment may be placed in a shielded enclosure for the purpose of minimizing uncontrolled extraneous fields from the environment in the region where the sample is placed.

In one embodiment of the shielding, the transduction equipment is located within a much larger enclosure, a least 3 times larger than the transduction equipment. This large container is lined with copper mesh attached to Earth ground. Such a container is commonly called a "Faraday cage". The copper mesh attenuates external environmental electromagnetic signals that are greater than approximately 10 kHz.

In a second embodiment of the shielding, the transduction equipment is located within a large enclosure constructed of sheet aluminum or other solid conductor with minimal structural discontinuities. Such a container attenuates external environmental electromagnetic signals that are greater than approximately 1 kHz.

In a third embodiment of the shielding, the transduction equipment is located within a very large set of three orthogonal Helmholtz coil pairs, at least 5 times larger than the transduction equipment. A fluxgate magnetic sensor container is located near the geometric center of the Helmholtz coil pairs, and somewhat distant from the transduction equipment. Signal from the fluxgate sensor is input to a feedback device, such as a Lindgren, Inc. Magnetic Compensation System, and a feedback current used to drive the Helmholtz coils, forcing a region within the Helmholtz coils to be driven to zero field. Since the Helmholtz coil pairs are very large, this region is also correspondingly large. Furthermore, since the transduction equipment uses relatively small coils, their field does not extend outward sufficiently to interfere with the fluxgate sensor. Such a set of Helmholtz coil pairs attenuates external environmental electromagnetic signals between 0.001 Hz and 1 kHz.

In a fourth embodiment of the shielding, the transduction equipment may be located in either a copper mesh or aluminum enclosure as mentioned above, and that enclosure itself located within the set of Helmholtz coil pairs mentioned above. Such a configuration can attenuate external environmental electromagnetic signals over their combined ranges.

in operation, the sample, e.g., an in vitro system or a mammalian subject or a selected target area of a mammalian subject, is placed centrally within the coils of the transduction equipment. Thus, for example, the coils may be at opposite ends of a support bed, or on opposite sides of the bed, and on opposite sides of the patient's head. The coil is then activated, using signal generation equipment like that shown in Figure 15, for transducing the system with agent-specific time-domain signals, preferably selected by one of the scoring algorithms described in Section III.

The transduction parameters, i.e., the selected transduction conditions to which the system is exposed are (i) the voltage of the applied time-domain signal, (ii) the duration of applied signal, and (iii) the scheduling of the applied signal. The applied voltage may be over a range from slightly greater than zero to up to about 100 Volts. The time of application may be from a few minutes to up to several days. The scheduling refers to the alternating periods of signal off and signal on, where these alternating periods can be quite brief, e.g., only a few second, where the signal is rapidly alternating between on and off conditions, to expended periods, for example, several hours on and several hours off.

As will be seen below, and in accordance with one aspect of the invention, optimal effector time-domain signals, and optimized transduction conditions for transducing a mammalian system can be identified by transduction studies with a simplified *in vitro* analog of the mammalian system.


V. <u>Method for generating a time-domain signal capable of producing an agent-specific effect on a mammalian system</u>

Sections II and III above describe methods for generating low-frequency, time-domain signals for an agent, e.g., compound known to act as an effector in an in vitro or mammalian system, and for selecting optimal time-domain signals from among those recorded. Briefly, as detailed in Section II, an agent capable of effecting a mammalian system is placed in a magnetically and electromagnetically shielded sample container, a selected magnetic-field stimulus is applied to the sample, e.g., by a Helmholtz coil surrounding the sample container, and a low-frequency, time-domain signal composed of sample source radiation superimposed on the injected stimulus magnetic field is recorded, e.g., by a SQUID in a cryogenic container, and signal recordings are made at each of a plurality of different stimulus magnetic field conditions, e.g., different noise or offset voltages. Typically between about 50 and 1,000 time-domain signals will provide an adequate set of signals from which optimized signals for transducing a mammalian system can be found. For example, the signal might be recorded at each of 50 different magnetic-field stimulus conditions, at each of ten different sample concentrations to produce 500 time-domain signals.

The plurality of low-frequency time-domain signals recorded for the agent at different magnetic-stimulus condition, and optionally, at different sample concentrations, are then analyzed by a scoring algorithm that measures the number of low-frequency components above a given threshold in a recorded signal, employing one of the scoring algorithms described in Section III above. In this step, each time-domain signal, representing a recording at a different selected magnetic-field stimulus is scored, and those signals having the highest score-meaning the highest number of low-frequency components above a given threshold in a recorded signal-are identified as candidates from which optimal signals capable of transducing an *in vitro* system can be identified. Typically, between 3-10 signals having the highest scores are identified by this scoring method.

In accordance with one aspect of the invention, the time-domain signals identified by the scoring algorithm above may be further selected for effectiveness in a mammalian system by testing each of the high-scoring signals in an *in vitro* system designed to serve as a simplified model that mirrors the interaction of the agent with a biochemical target in a more complex mammalian system. As noted above, the *in vitro* system may also be useful for identifying optimal transduction parameters, including the signal voltage applied to the transduction coils, the transduction time, and scheduling of system exposure to the transduction signal.

In one example that has been the focus of a number of studies, the effector agent is taxol (also known as paditaxel), an anti-tumor agent that is known is act by stimulating and stabilizing tubulin assembly into microtubules. In *vivo,* taxol interferes with cellular microtubule dynamics, causing cells to arrest in mitosis, interrupts intracellular transport of cargo, disrupts cell shape, cell motility and distribution of molecules on cell membranes. Thus, the ability of taxol to promote assembly of tubulin *in vitro* is directly related to its mechanism of action in vivo.

The *in vitro* test for selecting the most effective taxol-related time-domain signal that was chosen was a standard tubulin aggregation assay used for determining the tubulin assembly activity of an added compound. This assay has been described, for example, in Shelanski, M. L., Gaskin, F. and Cantor, C. R. (1973). Microtubule assembly in the absence of added nucleotides. Proc. Natl. Acad. Sci. U.S.A. 70, 765-768; and Lee, J. C. and Timasheff, S. N. (1977). In vitro reconstitution of calf brain microtubules: effects of solution variable. Biochemistry, 16, 1754-1762.

The assay protocol was designed for performing a single assay in a cuvette using a spectrophotometer set at 340 nm in a kinetic mode. HTS-Tubulin was purchased from Cytoskeleton, Inc. and is supplied in multiple vials as lyophilized protein. The lyophilized tubulin was resuspended in tubulin polymerization buffer (GPEM) to a final concentration of 1.5mg/ml. Before starting the assay, the spectrophotometer is set in a Kinetic Mode. Using a blank of tubulin polymerization buffer, the spectrophotometer is zeroed at 340nm. During the assay the data is collected either every 10secs, 30secs or 60secs as needed. Averaging time was set to 1 sec. In the studies reported below, the assay was carried out for 20 minutes.

in one group of *in vitro* tests, taxol-specific time-domain signals were obtained by recording low-frequency signals from a sample of taxol suspended in Cremophore™ to a final concentration of 6 mg/ml. The signals were recorded with injected DC offset, at noise level settings between 10 and 241 mV and in increments of 1 mV. A total of 241 time-domain signals over this injected-noise level range were obtained, and these were analyzed by the FFT autocorrelation algorithm detailed above, yielding 8 time-domain taxol signals for further *in vitro* testing. One of these, designated signal M2(3) was among the most effective of the 8 signals in the *in vitro* transduction studies described below.

The tubulin-assembly reaction was carried out by exposing the tubulin in GPEM buffer, at a concentration of 1.5 mg/ml to the following polymerization conditions: (i) a buffer (control), (ii) tubulin alone (2nd control); (iii) taxol, added to a final concentration of 4 $\mu$M, and (iv) the M2(3) taxol signal from above, transduced over a 20 minute period at a transduction voltage calculated to produce an approximately 1.693294 mG magnetic field. Change in optical absorption at 340 nm was measured continuously for each sample, and the OD340 data was used to calculate a rate of tubulin polymerization (dA/minute at 340 nm) at each one minute interval during the transduction study.

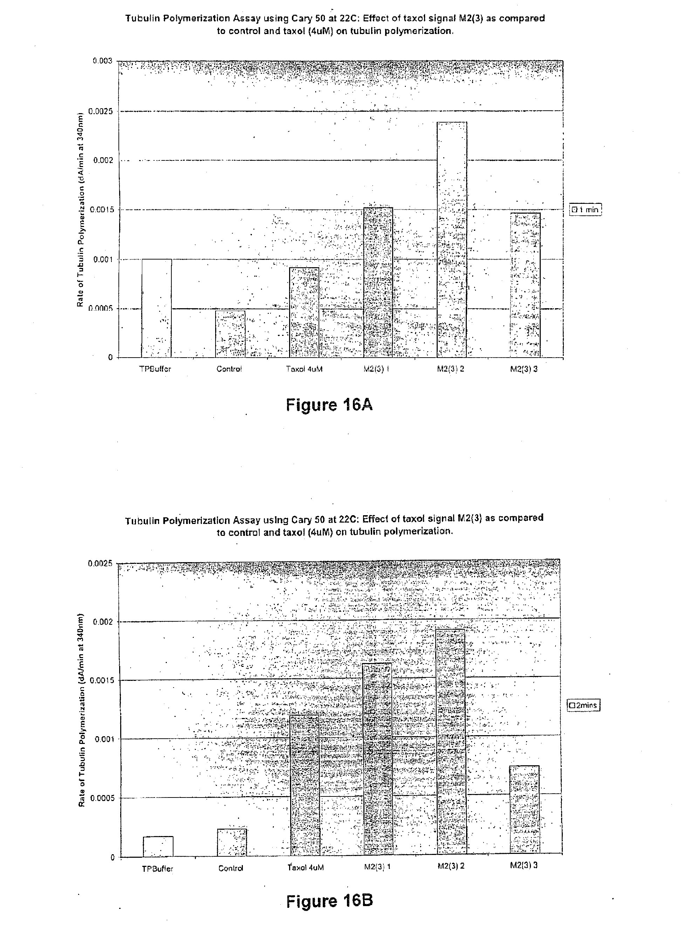

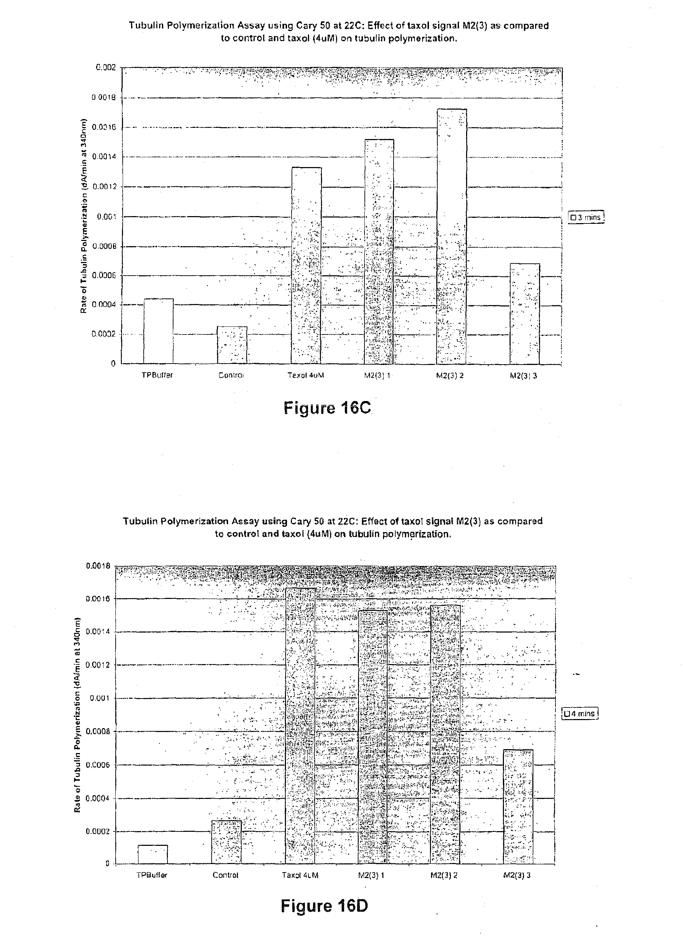

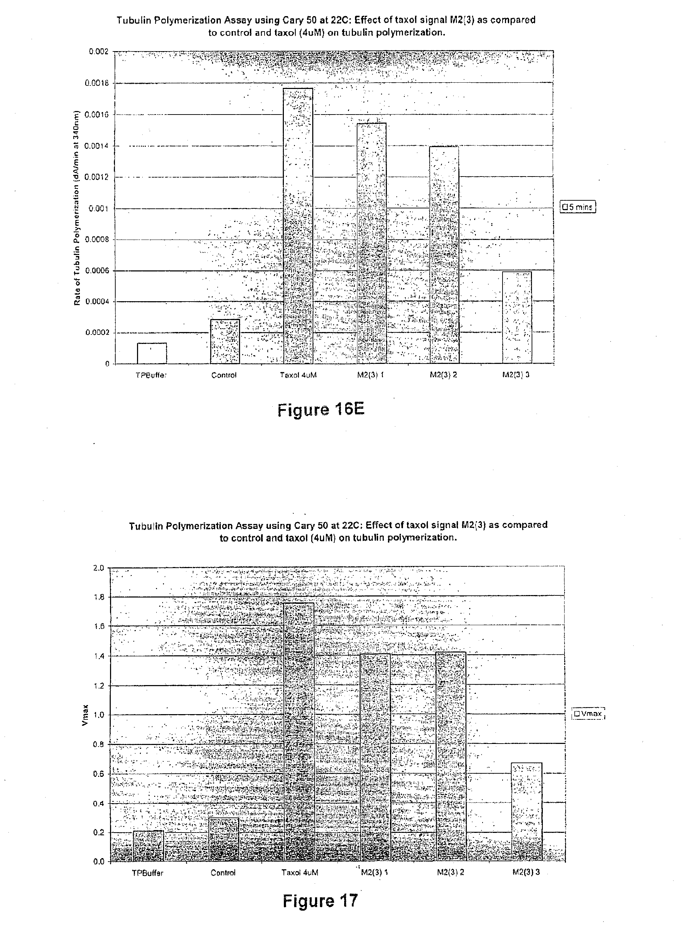

The results of the study, expressed as rate or tubulin polymerization at time points 1, 2, 3, 4 and 5, minutes, are shown in the bar graphs in Figures 10A-10F, respectively. In each figures M2(3)1, M2(3)2, and M2(3)3 represent the same signal, but transduced in separate transduction chambers under slightly different magnetic-field levels. The data show that, even after only one minute, the M2(3) transduction signal is effective to produce a significant increase in tubulin polymerization rate relative to the two control, and even with when compared with taxol itself (Figure 16A). At two minutes (Figure 16B), the rates of polymerization of two of the three M2(3) signals increased significantly, as was true for the taxol-containing sample as well. These trends continued for time periods 3, 4, and 5, minutes, with the rate of polymerization due to taxol itself overtaking the rate of tubulin polymerization by signal transduction at 5 minutes.

The Vmax of the reaction was also calculated for each sample at the end of the 20 minute assay period. Although the tubulin polymerization assay is known to involve three separate events, each with different Vmax values, a single composite Vmax value, representing the maximum rate of reaction over the entire assay is determined, and these values are plotted in Fig. 17. As seen, the control Vmax values were both between about 0.2 and 0.3. Taxol, at a concentration of 4 $\mu$M, showed the highest $V_{max}$ value, nearly 1.8, but two of the taxol-signal samples were also high, slightly above 1.4. The third taxol-signal sample was significantly lower, but still substantially above the control values.

A number of similar studies were carried out with transduction by other taxol time-domain signals generated and identified according to the methods detailed above. The results obtained were similar to those described above, and although variations in the extent of tubulin polymerization have been observed from signal to signal, and for the same signal at different times, the same degree of variation was also observed for taxol itself as the polymerizing agent. In addition, exposing the tubulin sample to white noise did not produce tubulin assembly activity statistically above control levels..

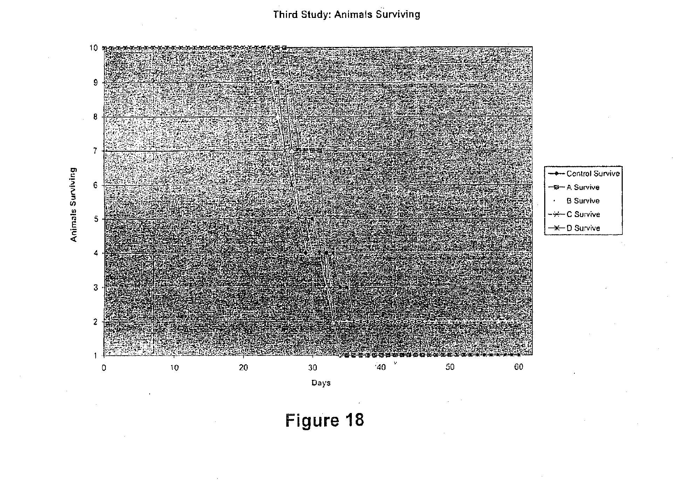

Based on the results from above, time-domain signal M2(3), and three additional taxol-specific low-frequency time-domain signals that also showed good activity in the tubulin in vitro assay were selected for transduction studies in a mammalian system. The signals are identified in Figure 18 as signals A, B, C, and D (the M2(3) signal). In this study, five groups of 10 mice each were each injected in the right frontal lobe with 5x10$^5$ U87 glioblastoma cells, and treatment with taxol signals was begun one day later. The transduction device used in this study was a 2-ft diameter right-angle cylinder with coil windings. These cylinders accommodate a standard mouse or rat cage so that mice are constantly exposed to the MIDS playback of signals. During the treatment, all ten mice in each group are housed in one cage and kept within the area of the central cylindrical cavity of the large transduction coil under continuous playback, while they are fed and watered. This results in a continuous exposure duty time of about 90-95% of the study duration of 60 days. The treatment involved either no signal or one or the four taxol-specific signals, applied to the coil by continuously sweeping the signal over a magnetic-field of between 80-110 G, and sweep frequency of 1 sweep/sec, over the entire course of the study. That is, each signal is played continuously to each of ten animals in a group, by sweeping the signal over a selected magnetic-field range, with only occasional interruption for cleaning and feeding.

The results of the study, plotted as number of animals surviving in each group over the 60 day period, are plotted in Figure 18. The effect of taxol alone (the compound) was not examined in the study since it is known to be poorly deliver to the brain, presumably because of inability to pass the blood brain barrier effectively. As seen, all ten of the animals in the control group died by day 34, and the same survival rate was obtained for treatment by taxol signal A. However, both taxol signal D, the time-domain signal shown above to promote tubulin assembly in the tubulin polymerization assay, and taxol signal B gave significantly improved survival times: mice in the signal-D group showed a 20% survival rate out to day 46, and mice in the signal-B group gave showed a final 20% survival rate.

The results demonstrate that low-frequency time-domain signals, when generated under a variety of selected magnetic-field injection conditions, and selected by a scoring algorithm, then further selected in an *in vitro* system that mimics the mechanism of action of the agent in a mammalian system, can be used to mimic the effect of the agent itself on the mammalian system.

The system for generating and selecting effective time-domain signals has been illustrated above with respect to taxol and an *in vitro* tubulin assembly assay, for selecting signals for producing a taxol-related anti-tumor effect by signal transduction. It will be appreciated that a variety of drugs used in treating mammalian diseases have identified drug targets that can be modeled in an *in vitro* system to identify drug-generated low-frequency time-domain signals that will be effective in producing a similar drug-target interaction in a mammalian host.

For example, a number of drugs that act to bind tubulin can be similar tested for optimized time-domain signals by a similar *in vitro* tubulin assay. Such drugs include, in addition to taxol, Docetaxel (Taxotere), Epothilones, Discodermolide, Colchicine, Combretastatins, 2-Methoxyestradiol, Methoxybenzene-sulphonamide Estramustine, and the vinca alkaloids, including Vinblastine (Velban), Vincristine (Oncovin), Vinorelbine (Navelbine), Vinflunine, Cryptophycin, Halichondrins, Dolastatins, and Hemiasterlins,

As another example, a large number of drugs function through their ability to bind to specific cell receptors, e.g., G protein receptors. For purposes of *in vitro* testing, there are many different mammalian cells, often with a genetically altered genome designed for allowing detection of agent binding to the target receptor, e.g., through the expression of a recombinant fluorescent protein, that can be cultured under conditions that would allow for the effects of signal transduction of the cells to be observed. Thus, in this treatment model, the transducing agent is the receptor-binding molecule, the *in vitro* system is a cell-culture system that is responsive to agent binding to produce a detectable cellular response, and the mammalian system is a mammalian subject having a disease state that is amenable to treatment by the binding agent.

Similarly, a number of drugs function through their ability to inhibit the activity of a soluble or membrane-associated enzyme. For *in vitro* testing, the target enzyme is likely to be adaptable to an *in vitro* enzyme reaction assay in which a drug effect on the activity of the enzyme can be detected, e.g., colorometrically, as an increase or decrease in enzyme activity with respect to a detectable substrate. Thus, in this treatment model, the transducing agent is the enzyme binding agent, the *in vitro* system is an enzyme assay reaction which is responsive to agent to produce a detectable change in enzyme kinetics, and the mammalian system is a mammalian subject having a disease state normally treated by the binding agent.

VI. Forming a transduction NMR signal

in one embodiment of the invention, the low-frequency signal that is used to transduce a biological system (see below) is a low-frequency NMR spectral signal of the transducing agent, e.g., therapeutic agent.

An NMR spectrum, e.g., conventional high-field NMR signal, consists of a series of frequency bands that is characteristic of a sample solution when placed in a static magnetic field of fixed magnitude. In proton NMR, the Larmor frequency of hydrogen nuclei is split into the frequency bands by spin coupling processes and local shielding effects that occur within the sample molecule. For hydrogen nuclei, the gyromagnetic ratio is 42.58 MHz/T, and thus in a typical NMR machine of 7 T, the Larmor frequency is (42.58 MHz/T) * (7 T) = 300 MHz, split into bands that are several Hz apart. For hydrogen nuclei in a much weaker field of 7 uT, the Larmor frequency is (42.58 MHz/T) * (7 uT) = 300 Hz, again split into bands that are several Hz apart. Thus, if the field magnitude is reduced, the frequency bands shift downwards by a corresponding amount, but the splitting (from spin coupling processes) remains the same. Any local shielding effects become negligible at lower field strengths.

To calculate the NMR spectrum at a low field strength using data obtained in a high-field instrument, the following operations are carried out. Assume, for example, that a high-field NMR instrument yields three peaks at 1.03 ppm, 1.13 ppm, and 1.23 ppm in a 7 T magnetic field, due to spin-coupling processes within a methyl group on a molecule, and that a TMS standard yields a peak at 0.00 ppm. The frequencies of the methyl bands relative to the TMS standard would be the ppm shift difference multiplied by the Larmor frequency:

$$(1.03 \text{ ppm} - 0.00 \text{ ppm}) * 300000000 \text{ Hz} = 310 \text{ Hz}$$

$$(1.13 \text{ ppm} - 0.00 \text{ ppm}) * 300000000 \text{ Hz} = 340 \text{ Hz}$$

$$(1.23 \text{ ppm} - 0.00 \text{ ppm}) * 300000000 \text{ Hz} = 370 \text{ Hz}$$

Since TMS theoretically has no significant chemical shift, the location of its peak will be the simple Larmor frequency of 300000000 Hz. Thus, the actual frequencies of the methyl group are:

$$300000000 \text{ Hz} + 310 \text{ Hz} = 300000310 \text{ Hz}$$

$$300000000 \text{ Hz} + 340 \text{ Hz} = 300000340 \text{ Hz}$$

$$300000000 \text{ Hz} + 370 \text{ Hz} = 300000370 \text{ Hz}$$

If there were no chemical shift effect (such as in a low magnetic field), then the frequencies of the methyl group would be centered on the Larmor frequency, no longer shifted away from it. Thus, the methyl middle band of would be centered on the Larmor frequency, and the two side bands would be at +30 Hz and -30 Hz relative to the Larmor frequency:

$$370 \text{ Hz} - 340 \text{ Hz} = +30 \text{ Hz}$$

$$310 \text{ Hz} - 340 \text{ Hz} = -30 \text{ Hz}$$

The Larmor frequency in a 7 microTesla field is (42.58 MHz/T) * (7 uT) = 300 Hz. Thus, the methyl group should exhibit a middle band at the Larmor frequency, 300 Hz, and two side bands at 300 Hz + 30 Hz = 330 Hz and 300 Hz - 30 Hz = 270 Hz. The net spectrum of the methyl group would then be 330 Hz, 300 Hz, and 270 Hz.

This is the calculated NMR spectrum that should theoretically occur in a 7 uT magnetic field. Note that this calculation is only valid for low field strengths that are not so low that the final frequencies are negative.

As an alternative approach, a low-frequency NMR signal of a given agent may be generated directly by low-field NMR detection at millitesla magnetic fields, and using an untuned superconducting quantum interference device (SQUID) magnetometer (see above) to detect the magnetic signals. That is, the signal-generating apparatus described above, but operated in a low-field NMR mode, may be employed to directly generate the low-field NMR signal.

Once the low-field NMR signal is calculated or generated, the transduction signal may be constructed. This can be done by calculating the inverse Fourier transform of the low-field NMR spectrum, to generate time series data. This time series data can also be generated by adding together a set of sine waves having the same frequencies and amplitudes given in the low-field NMR spectrum. This time series data can then be used to control the voltage of a suitable voltage generator. This time-varying voltage can then be applied across a Helmholtz coil, whereby electrical current flows through the conductor and generates a time-varying magnetic field. This time-varying magnetic field is then used for biological transduction.

In another embodiment of the invention, EPR (Electron Paramagnetic Resonance) signals are used instead of NMR (Nuclear Magnetic Resonance) signals. EPR involves electron spin - nuclear spin interactions, whereas NMR involves nuclear spin - nuclear spin interactions. The procedures outlined in this application for NMR data are functionally equivalent to the procedures to be used for EPR data, except that EPR data is typically at somewhat higher frequencies.

The above detailed description of embodiments of the invention is not intended to be exhaustive or to limit the invention to the precise form disclosed above. While specific embodiments of, and examples for, the invention are described above for illustrative purposes, various equivalent modifications are possible within the scope of the invention, as those skilled in the relevant art will recognize. For example, while processes or blocks are presented in a given order, alternative embodiments may perform routines having steps, or employ systems having blocks, in a different order, and some processes or blocks may be deleted, moved, added, subdivided, combined, and/or modified. Each of these processes or blocks may be implemented in a variety of different ways. Also, while processes or blocks are at times shown as being performed in series, these processes or blocks may instead be performed in parallel, or may be performed at different times.

The teachings of the invention provided herein can be applied to other systems, not necessarily the system described above. The elements and acts of the various embodiments described above can be combined to provide further embodiments.

All of the above patents and applications and other references, including any that may be listed in accompanying filing papers, are incorporated herein by reference. Aspects of the invention can be modified, if necessary, to employ the systems, functions, and concepts of the various references described above to provide yet further embodiments of the invention.

These and other changes can be made to the invention in light of the above Detailed Description. While the above description details certain embodiments of the invention and describes the best mode contemplated, no matter how detailed the above appears in text, the invention can be practiced in many ways. Details of the signal processing system may vary considerably in its implementation details, while still being encompassed by the invention disclosed herein. As noted above, particular terminology used when describing certain features or aspects of the invention should not be taken to imply that the terminology is being redefined herein to be restricted to any specific characteristics, features, or aspects of the invention with which that terminology is associated. In general, the terms used in the following claims should not be construed to limit the invention to the specific embodiments disclosed in the specification, unless the above Detailed Description section explicitly defines such terms. Accordingly, the actual scope of the invention encompasses not only the disclosed embodiments, but also all equivalent ways of practicing or implementing the invention under the claims.

## Claims

1. A method for generating a signal capable of producing an agent-specific effect on a mammalian system, when the system is transduced by the signal within the environment of an electromagnetic tranducer, comprising:

   (a) placing a sample containing the agent in a sample container having both magnetic and electromagnetic shielding, wherein the sample acts as a signal source for low-frequency molecular signals, and wherein the magnetic shielding is external to a cryogenic container;
   (b) injecting a stimulus magnetic field into the sample, under a selected stimulus magnetic field condition,
   (c) recording a low-frequency, time-domain signal composed of sample source radiation superimposed on the injected stimulus magnetic field in the cryogenic container,
   (d) repeating steps (b) and (c) at each of a plurality of different stimulus magnetic field conditions,
   (e) identifying from among the signals recorded in step (c), one or more signals having the highest signal scores when analyzed by a scoring algorithm that measures the number of low-frequency components above a given threshold in a recorded signal,
   (f) testing each signal identified in step (e) for its ability to produce an agent-specific response in a *in vitro* system containing components that are responsive to the agent, when the *in vitro* system is transduced with the signal within the environment of an electromagnetic tranducer, and
   (g) selecting one or more signals that produce the greatest agent-specific transduction effect in the *in vitro* system.

2. The method of claim 1, wherein the different conditions of stimulus magnetic field include conditions selected from the group consisting of:

   (i) white noise, injected at voltage level calculated to produce a selected magnetic field at the sample of between 0 and 1G (Gauss), (ii) a DC offset, injected at voltage level calculated to produce a selected magnetic field at the sample of between 0 and 1 G, and (iii) sweeps over a low-frequency range, injected successively over a sweep range between at least about 0-1 kHz, and at an injected voltage calculated to produce a selected magnetic field at the sample of between 0 and 1 G.

3. The method of claim 1, wherein step (f) further includes, after testing a time-domain signal for its ability to produce an agent-specific response in a *in vitro* system containing components that are responsive to the agent, testing the ability of signal to produce an agent-specific response under varying transduction conditions, including variations in transduction voltage applied within the environment of an electromagnetic tranducer, thus to optimize transduction conditions for transduction in the mammalian system.

4. The method of claim 1, wherein step (e) is carried out by a method selected from the group consisting of:

(i) autocorrelating the time domain signal, generating an FFT (Fast Fourier Transform) of the autocorrelated signal over a selected frequency range within the range DC to 8kHz, assigning to the FFT signal a score related to a number of peaks above a mean average noise value, and selecting a time-domain signal based on the score;
(ii) calculating a pair of phase spaces for two time domain signals, and performing a mathematical comparison to provide a measure of difference between the two;
(iii) generating a histogram that shows, for each event bin f over a selected frequency range within a range DC to 8kHz, a number of event counts in each bin, where f is a sampling rate for sampling the time domain signal, assigning to the histogram, a score related to number of bins that are above a given threshold; and selecting a time-domain signal based on the score;
(iv) cross-correlating a small block of data near the beginning of the time domain signal with the remainder of the time series, and counting the occurrences that the resulting cross-correlation surpasses a given threshold; and
(v) calculating a series of Fourier spectra of the time-domain signal over each of multiple defined time periods, in a selected frequency range between DC and 8 kHz, averaging the Fourier spectra; assigning to the averaged FFT signal a score related to the number of peaks above a mean average noise value, and selecting a time-domain signal based on the score.

5. The method of claim 1, wherein the electromagnetic transducer includes a Helmholtz coil having a pair of aligned electromagnetic coils defining an exposure station therebetween, constituting the environment of the electromagnetic environment, and step (f) includes placing the *in vitro* system within the aligned coils, and transducing the system with an agent-specific time-domain signal identified in step (e).

6. The method of claim 1, wherein the agent is an anti-neoplastic drug effective to promote tubulin aggregation in a cell-free *in vitro* system, and step (f) includes placing a tubulin-containing composition within the environment of the electromagnetic transducer, and transducing the composition with an agent-specific time-domain signal identified in step (e).

7. Apparatus for producing low-frequency, time-domain signals that are candidates for transducing an *in vitro* or mammalian system that is responsive to the presence of a selected agent, comprising

(a) a container adapted for receiving a sample of an agent, the container having both magnetic and electromagnetic shielding;
(b) an adjustable-power source operable to inject a stimulus magnetic field into the container, with a sample in the container, at each of a plurality of selected stimulus magnetic field conditions selected from the group consisting of (i)white noise, injected at voltage level calculated to produce a selected magnetic field at the sample of between 0 and 1 G (Gauss), (ii) a DC offset, injected at voltage level calculated to produce a selected magnetic field at the sample of between 0 and 1 G, and (iii) sweeps over a low-frequency range, injected successively over a sweep range between at least about 0-1 kHz, and at an injected voltage calculated to produce a selected magnetic field at the sample of between 0 and 1 G,
(c) a detector for recording, at each of the different stimulus magnetic field conditions injected by said power source, (b) the electromagnetic time-domain signals composed of sample source radiation superimposed on the injected stimulus magnetic fields,
(d) a memory device for storing the signals recorded by the detector, and
(e) a computer operable to:
(i) retrieve time-domain signals stored in the memory device;
(ii) analyzing the retrieved time-domain signals by a scoring algorithm that measures the number of low-frequency components above a given threshold in a recorded signal, and
(iii) identifying those time-domain signals having the greatest number of low-frequency components above the threshold.

8. The apparatus of claim 7, wherein the container is an attenuation tube having a sample-holding region, a magnetic shielding cage surrounding the region, and a Faraday cage contained within the magnetic shielding cage and also surrounding the region, the source of a Gaussian noise includes a Gaussian noise generator and a Helmholtz coil which is contained within the magnetic cage and the Faraday cage, and which receives a noise output signal from the noise generator, and which further includes, for use in removing stationary noise components in the time-dependent signal, a signal inverter operatively connected to the noise source and to the SQUID (Superconducting QUantum Interference Device), for receiving Gaussian noise from the noise source and outputting into the SQUID, Gaussian noise in inverted form with respect to the Gaussian noise injected into the sample.

9. The apparatus of claim 7, wherein said computer, in analyzing the retrieved time-domain signals is operable to apply an analysis algorithm selected from the group consisting of:

> (i) autocorrelating the time domain signal, generating an FFT (Fast Fourier Transform) of the autocorrelated signal over a selected frequency range within the range DC to 8kHz, assigning to the FFT signal a score related to a number of peaks above a mean average noise value, and selecting a time-domain signal based on the score;
> (ii) calculating a pair of phase spaces for two time domain signals, and performing a mathematical comparison to provide a measure of difference between the two;
> (iii) generating a histogram that shows, for each event bin f over a selected frequency range within a range DC to 8kHz, a number of event counts in each bin, where f is a sampling rate for sampling the time domain signal, assigning to the histogram, a score related to number of bins that are above a given threshold; and selecting a time-domain signal based on the score;
> (iv) cross-correlating a small block of data near the beginning of the time domain signal with the remainder of the time series, and counting the occurrences that the resulting cross-correlation surpasses a given threshold; and
> (v) calculating a series of Fourier spectra of the time-domain signal over each of multiple defined time periods, in a selected frequency range between DC and 8 kHz, averaging the Fourier spectra; assigning to the averaged FFT signal a score related to the number of peaks above a mean average noise value, and selecting a time-domain signal based on the score.

10. A system for producing an agent-specific effect on a mammalian system comprising,

> (1) a storage medium having stored thereon, an agent-specific low-frequency time-domain signal produced by the steps of:

>> (a) placing a sample to which the mammalian system is responsive in a sample container having both magnetic and electromagnetic shielding, wherein the sample acts as a signal source for low-frequency molecular signals, and wherein the magnetic shielding is external to a cryogenic container;
>> (b) injecting a stimulus magnetic field into the sample, under a selected stimulus magnetic field condition,
>> (c) recording a low-frequency, time-domain signal composed of sample source radiation superimposed on the injected stimulus magnetic field in the cryogenic container,
>> (d) repeating steps (b) and (c) at each of a plurality of different stimulus magnetic field conditions,
>> (e) identifying from among the signals recorded in step (c), one or more signals having the highest signal scores when analyzed by a scoring algorithm that measures the number of low-frequency components above a given threshold in a recorded signal,
>> (f) testing each signal identified in step (e) for its ability to produce an agent-specific response in a *in vitro* system containing components that are responsive to the agent, when the *in vitro* system is transduced by the signal within the environment of an electromagnetic transducer,

> (2) an electromagnetic transducer composed of one or more electromagnetic coils, said coils having an interior region defining a transducer environment in which the sample is received, and
> (3) an amplifier for amplifying the signal received from the storage medium and supplying the amplified signal to the transduction coil(s).

11. The apparatus of claim 10, wherein the different conditions of stimulus magnetic field used in producing the agent-specific low-frequency time-domain signal are selected from the group consisting of:

> (i) white noise, injected at voltage level calculated to produce a selected magnetic field at the sample of between 0 and 1 G (Gauss), (ii) a DC offset, injected at voltage level calculated to produce a selected magnetic field at the sample of between 0 and 1 G, and (iii) sweeps over a low-frequency range, injected successively over a sweep range between at least about 0-1 kHz, and at an injected voltage calculated to produce a selected magnetic field at the sample of between 0 and 1 G,.

**12.** The apparatus of claim 10, wherein the electromagnetic transducer includes a Helmholtz coil having a pair of aligned electromagnetic coils defining an interior region therebetween.

**13.** A storage medium having stored thereon, an agent-specific low-frequency time-domain signal produced by the steps of:

(a) placing a sample to which the mammalian system is responsive in a sample container having both magnetic and electromagnetic shielding, wherein the sample acts as a signal source for low-frequency molecular signals, and wherein the magnetic shielding is external to a cryogenic container;
(b) injecting a stimulus magnetic field into the sample, under a selected stimulus magnetic field condition,
(c) recording a low-frequency, time-domain signal composed of sample source radiation superimposed on the injected stimulus magnetic field in the cryogenic container,
(d) repeating steps (b) and (c) at each of a plurality of different stimulus magnetic field conditions,
(e) identifying from among the signals recorded in step (c), one or more signals having the highest signal scores when analyzed by a scoring algorithm that measures the number of low-frequency components above a given threshold in a recorded signal,
(f) testing each signal identified in step (e) for its ability to produce an agent-specific response in a *in vitro* system containing components that are responsive to the agent, when the *in vitro* system is transduced by the signal within the environment of an electromagnetic transducer.

**14.** The storage medium of claim 13, wherein the different conditions of stimulus magnetic field used in producing the agent-specific low-frequency time-domain signal are selected from the group consisting of:

a DC offset, injected at a voltage calculated to produce a selected magnetic field at the sample of between 0 and 1 G; and
successive sweeps over a low-frequency range between at least about 0-1kHz, injected at a sweep voltage calculated to produce a selected magnetic field at the sample of between 0 and 1 G

**15.** The storage medium of claim 13, wherein the agent-specific, time-domain signal is generated from a sample of an anti-neoplastic agent effective to promote tubulin aggregation in an in vitro system.

FIG. 1

FIG. 2

FIG. 3

FIG. 4

Processing Unit — 704

700

721

722

710 · 720 · 711 · 702

726 · 711 · 724 · 710 · 728

708 · 712

716

718

**FIG. 5**

*FIG. 6*

**FIG. 7**

Start

Place appropriate sample in sample chamber — 300

Inject white Gaussian noise — 301

Detect sample emission and injected noise — 302

Remove injected noise, optional — 304

Amplification of detected signal — 306

A/D conversion and storage — 308

Fourth data run completed for a given sample? — 310

No

Yes

Lowpass filtering — 312

Bandpass filtering — 314

Fourier transform — 316

Correlation and comparison — 318

Output sample's emission spectrum — 320

*FIG. 8*

*FIG. 9*

(SampleDuration *
SampleRate) ==
SampleCount data
samples — 2102

FFTSize = 8192;
n = SampleCount /
FFTSize * 2
m = 0; — 2104

2113 — initialize histogram and
average arrays to zero

2108

FFTSize bins
for histogram

2110

FFTSize bins
for average
power

2114 — copy FFTSize samples
of WAV data into buffer
starting at
m * FFTSize / 2

FFTSize
copied
samples — 2112

perform RFFT on copied
samples — 2115

normalize fft output — 2116

compute avg power for
all FFTSize — 2117

add normalized value to
average power slot m — 2118

foreach FFTSize , if
power > avg * epsilon
add one to histotgram
slot m — 2119

m = m + 1 — 2120

Yes

m < n ? — No →  divide each
average power
bin by n — 2122 → Display average
power and
histogram — 2123

2121

*FIG. 10*

| 406 | | 402 | | 404 |
|-----|--|-----|--|-----|
| Increment noise level | → | Sample time domain signals | ← | Set initial noise level |

408
Autocorrelate time domain signals

410
Generate FFT

412
Score all peaks |p|>average noise level

414
Calculate score

416
Score increasing?

Y

N

418
Second peak score

420
Select TDS with peak score

422
Time domain signals

*FIG. 11*

437
Increment noise level

424
Sample time domain signals

426
Set initial noise level

428
Generate series of FFT for each signal

430
Average FFTs

432
Score all peaks |p|>average noise level

434
Calculate score

436
Score increasing?

Y

N

438
Second peak score

440
Select TDS with peak score

442
Time domain signals

*FIG. 12*

Figure 13

Diagram 1

Transduction Equipment Layout

Figure 14

Diagram 2a
Coil and Box Layout

Figure 15

Tubulin Polymerization Assay using Cary 50 at 22C: Effect of taxol signal M2(3) as compared
to control and taxol (4uM) on tubulin polymerization.

Figure 16A

Tubulin Polymerization Assay using Cary 50 at 22C: Effect of taxol signal M2(3) as compared
to control and taxol (4uM) on tubulin polymerization.

Figure 16B

Tubulin Polymerization Assay using Cary 50 at 22C: Effect of taxol signal M2(3) as compared to control and taxol (4uM) on tubulin polymerization.

**Figure 16C**

Tubulin Polymerization Assay using Cary 50 at 22C: Effect of taxol signal M2(3) as compared to control and taxol (4uM) on tubulin polymerization.

**Figure 16D**

Tubulin Polymerization Assay using Cary 50 at 22C: Effect of taxol signal M2(3) as compared to control and taxol (4uM) on tubulin polymerization.

Figure 16E

Tubulin Polymerization Assay using Cary 50 at 22C: Effect of taxol signal M2(3) as compared to control and taxol (4uM) on tubulin polymerization.

Figure 17

Third Study: Animals Surviving

Figure 18

Europäisches
Patentamt

European
Patent Office

Office européen
des brevets

## EUROPEAN SEARCH REPORT

**Application Number**

EP 11 15 6208

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ATKINS P W: "Physical Chemistry , Chapter 16",<br>1 January 1990 (1990-01-01), Oxford University Press, Oxford, XP002324574,<br>ISBN: 0-19-855284-X<br>* page 458 - page 497 * | 1-15 | INV.<br>G01N37/00 |
| A | ATKINS P W: "Physical Chemistry, Chapter 18",<br>1990, OXFORD UNIVERSITY PRESS, OXFORD, XP002478451,<br>ISBN: 0-19-855284-X<br>* page 535 - page 563 * | 1-15 | |
| A | Hore P J: "Nuclear Magnetic Resonance", 1995, Oxford University Press, Oxford, XP002631029,<br>ISBN: 978-0-19-855682-4<br>* page 22 - page 43 * | 1-15 | |
| A | US 6 133 734 A (MCKEON DONALD C [US])<br>17 October 2000 (2000-10-17)<br>* the whole document * | 1-15 | TECHNICAL FIELDS SEARCHED (IPC)<br>G01N<br>G01V |
| A | US 6 586 931 B2 (TAICHER GERSH ZVI [US])<br>1 July 2003 (2003-07-01)<br>* the whole document * | 1-15 | |
| A | MAEHLE ANDREAS-HOLGER ET AL: "The emergence of the drug receptor theory", NATURE REVIEWS. DRUG DISCOVERY, NATURE PUBLISHING GROUP, GB,<br>vol. 1, no. 8, 1 August 2002 (2002-08-01), pages 637-641, XP007914858,<br>ISSN: 1474-1776, DOI: DOI:10.1038/NRD875<br>* the whole document * | 1-15 | |

-/--

The present search report has been drawn up for all claims

| Place of search | Date of completion of the search | Examiner |
|---|---|---|
| The Hague | 1 April 2011 | Wilhelm, Jörg |

EPO FORM 1503 03.82 (P04C01)

Europäisches
Patentamt

European
Patent Office

Office européen
des brevets

# EUROPEAN SEARCH REPORT

**Application Number**

EP 11 15 6208

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2006/015038 A (WAVBANK INC [US]; BUTTERS JOHN T [US]; BUTTERS BENNETT M [US]; NAUGHTO) 9 February 2006 (2006-02-09) * the whole document * ----- | 1-15 | |
| X | WO 99/54731 A (DIGIBIO S A [FR]; BENVENISTE JACQUES [FR]; GUILLONNET DIDIER [FR]) 28 October 1999 (1999-10-28) * the whole document * ----- | 1-15 | |
| A | BENVENISTE J ET AL: "QED AND DIGITAL BIOLOGY", RIVISTA DI BIOLOGIA, UNIVERSITA DEGLI STUDI, PERUGIA, IT, vol. 97, no. 1, 1 January 2004 (2004-01-01), pages 169-172, XP008059428, ISSN: 0035-6050 * the whole document * ----- | 1-15 | |

**TECHNICAL FIELDS SEARCHED (IPC)**

The present search report has been drawn up for all claims

| Place of search | Date of completion of the search | Examiner |
|---|---|---|
| The Hague | 1 April 2011 | Wilhelm, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&: member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 15 6208

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2011

| Patent document cited in search report | | Publication date | Patent family member(s) | | Publication date |
|---|---|---|---|---|---|
| US 6133734 | A | 17-10-2000 | NONE | | |
| US 6586931 | B2 | 01-07-2003 | CA | 2445034 A1 | 31-10-2002 |
| | | | EP | 1390775 A2 | 25-02-2004 |
| | | | GB | 2391947 A | 18-02-2004 |
| | | | NO | 20034637 A | 19-12-2003 |
| | | | WO | 02086541 A2 | 31-10-2002 |
| | | | US | 2002153887 A1 | 24-10-2002 |
| WO 2006015038 | A | 09-02-2006 | AU | 2005269345 A1 | 09-02-2006 |
| | | | AU | 2005323470 A1 | 13-07-2006 |
| | | | AU | 2010246466 A1 | 16-12-2010 |
| | | | BR | PI0512678 A | 01-04-2008 |
| | | | BR | PI0513910 A | 20-05-2008 |
| | | | CA | 2573350 A1 | 09-02-2006 |
| | | | CA | 2574616 A1 | 13-07-2006 |
| | | | CN | 101031796 A | 05-09-2007 |
| | | | EP | 1779122 A2 | 02-05-2007 |
| | | | EP | 1792179 A2 | 06-06-2007 |
| | | | JP | 2008508523 T | 21-03-2008 |
| | | | JP | 2008507977 T | 21-03-2008 |
| | | | US | 2009156659 A1 | 18-06-2009 |
| | | | US | 2007231872 A1 | 04-10-2007 |
| | | | WO | 2006073491 A2 | 13-07-2006 |
| WO 9954731 | A | 28-10-1999 | AU | 3336299 A | 08-11-1999 |
| | | | EP | 1073900 A1 | 07-02-2001 |
| | | | FR | 2777656 A1 | 22-10-1999 |

EPO FORM P0459

For more details about this annex : see Official Journal of the European Patent Office, No. 12/82

## REFERENCES CITED IN THE DESCRIPTION

### Patent documents cited in the description

- US 60593006 B

- US 60591549 B

### Non-patent literature cited in the description

- **SHELANSKI, M. L. ; GASKIN, F. ; CANTOR, C. R.** Microtubule assembly in the absence of added nucleotides. *Proc. Natl. Acad. Sci. U.S.A.,* 1973, vol. 70, 765-768

- **LEE, J. C. ; TIMASHEFF, S. N.** In vitro reconstitution of calf brain microtubules: effects of solution variable. *Biochemistry,* 1977, vol. 16, 1754-1762